# EUROPEAN PATENT SPECIFICATION

(11) **EP 3 418 272 B1**
(45) Date of publication and mention of the grant of the patent: **30.08.2023**
(21) Application number: 18178734.2
(22) Date of filing: 20.06.2018
(51) Int. Cl.: C07D 251/24, C07D 401/04, C07F 5/02, H10K 50/00

(54) **TRIAZINE COMPOUNDS AND ORGANIC LIGHT-EMITTING DEVICES INCLUDING THE SAME**
TRIAZIN-VERBINDUNGEN UND ORGANISCHE LICHTEMITTIERENDE VORRICHTUNGEN DAMIT
COMPOSÉS TRIAZINE ET DISPOSITIFS ÉLECTROLUMINESCENTS ORGANIQUES LES INCLUANT

(30) Priority: 21.06.2017 KR 20170078597; 10.01.2018 US 201815867357
(43) Date of publication of application: 26.12.2018
(73) Proprietor: Samsung Display Co., Ltd., Gyeonggi-do (KR)
(72) Inventor: Park, Junha, Yongin-si, Gyeonggi-do (KR); Sim, Munki, Yongin-si, Gyeonggi-do (KR); Lee, Hyoyoung, Yongin-si, Gyeonggi-do (KR); Kim, Youngkook, Yongin-si, Gyeonggi-do (KR); Hwang, Seokhwan, Yongin-si, Gyeonggi-do (KR)
(74) Representative: Gulde & Partner

(56) References cited:
- EP-A2- 3 246 963
- WO-A2-2004/106311
- US-A1- 2016 028 021
- US-A1- 2016 046 563
- US-A1- 2016 218 298
- US-A1- 2017 098 779
- DONG RYUN LEE ET AL: "Design Strategy for 25% External Quantum Efficiency in Green and Blue Thermally Activated Delayed Fluorescent Devices", ADVANCED MATERIALS, vol. 27, no. 39, 2015, pages 5861-5867, XP055328577, ISSN: 0935-9648, DOI: 10.1002/adma.201502053
- PRADIP DEBNATH ET AL: "A novel straightforward synthesis of 2,4,6-triaryl-1,3,5-triazines via copper-catalyzed cyclization of N-benzylbenzamidines", TETRAHEDRON LETTERS, vol. 55, no. 51, 2014, pages 6976-6978, XP055504920, ISSN: 0040-4039, DOI: 10.1016/j.tetlet.2014.10.091
- OLAF ZEIKA ET AL: "Synthesis of Polynitroxides Based on Nucleophilic Aromatic Substitution", ORGANIC LETTERS, vol. 12, no. 16, 2010, pages 3696-3699, XP055343403, ISSN: 1523-7060, DOI: 10.1021/ol101529u
- SAMBASIVARAO KOTHA ET AL: "Synthesis of nano-sized C3-symmetric 2,4,6-triphenyl-1,3,5-s-triazine and 1,3,5-triphenylbenzene derivatives via the trimerization followed by Suzuki-Miyaura cross-coupling or O-alkylation reactions and their biological evaluation", INDIAN JOURNAL OF CHEMISTRY, SECTION B, vol. 48B, no. 12, 2009, pages 1766-1770, XP055505358, ISSN: 0376-4699
- TSUTOMU ISHI-I ET AL: "High Electron Drift Mobility in an Amorphous Film of 2,4,6,-Tris[4-(1-naphthyl)phenyl]-1,3,5-tr iazine", CHEMISTRY LETTERS, vol. 33, no. 10, 2004, pages 1244-1245, XP055505217, ISSN: 0366-7022, DOI: 10.1246/cl.2004.1244
- RICARDA BERGER ET AL: "New symmetrically substituted 1,3,5-triazines as host compounds for channel-type inclusion formation", CRYSTENGCOMM, vol. 14, no. 3, 2012, pages 768-770, XP055505215, DOI: 10.1039/C2CE06034E

## Description

### BACKGROUND

### 1. Field

One or more embodiments relate to a heterocyclic compound and an organic light-emitting device including the same.

### 2. Description of the Related Art

Organic light-emitting devices include self-emission devices that have wide viewing angles, high contrast ratios, short response times, and excellent characteristics in terms of brightness, driving voltage, and response speed, as compared to other devices in the art.

An example of such an organic light-emitting device may include a first electrode disposed on a substrate, and a hole transport region, an emission layer, an electron transport region, and a second electrode, which are sequentially disposed on the first electrode. Holes provided from the first electrode may move toward the emission layer through the hole transport region, and electrons provided from the second electrode may move toward the emission layer through the electron transport region. Carriers, such as holes and electrons, recombine in the emission layer to produce excitons. These excitons transit (e.g., relax) from an excited state to a ground state, thereby generating light. Related patent literature may be found in US 2016/218298 A1, US 2016/028021 A1, EP 3 246 963 A2, US 2017/098779 A1, US 2016/046563 A1 and WO 2004/106311 A2, and in the following non-patent literature: Dong Ryun Lee et al. ("Design Strategy for 25% External Quantum Efficiency in Green and Blue Thermally Activated Delayed Fluorescent Devices", ADVANCED MATERIALS, vol. 27, no. 39, 2015, pages 5861-5867); Pradip Debnath et al. ("A novel straightforward synthesis of 2,4,6-triary1-1 ,3,5-triazines via copper-catalyzed cyclization of N-benzylbenzamidines", TETRAHEDRON LETTERS, vol. 55, no. 51, 2014, pages 6976-6978); Olaf Zeika et al. ("Synthesis of Polynitroxides Based on Nucleophilic Aromatic Substitution", ORGANIC LETTERS, vol. 12, no. 16, 2010, pages 3696-3699); Sambasivarao Kotha et al. ("Synthesis of nano-sized C3-symmetric 2,4,6-tripheny1-1,3,5-s-triazine and 1,3,5-triphenylbenzene derivatives via the trimerization followed by Suzuki-Miyaura cross-coupling or 0-alkylation reactions and their biological evaluation", INDIAN JOURNAL OF CHEMISTRY, SECTION B, vol. 48B, no. 12, 2009, pages 1766-1770); Tsutomu Ishi-I et al. ("High Electron Drift Mobility in an Amorphous Film of 2,4,6,-Tris[4-(1-naphthyl)pheny1]-1,3,5-triazine",CHEMISTRY LETTERS,vol. 33, no. 10, 2004, pages 1244-1245); and Ricarda Berger et al. ("New symmetrically substituted 1,3,5-triazines as host compounds for channel-type inclusion formation", CRYSTENGCOMM, vol. 14, no. 3, 2012, pages 768-770).

### SUMMARY

Aspects of embodiments of the present disclosure provide a novel heterocyclic compound and an organic light-emitting device including the same.

Additional aspects of embodiments will be set forth in part in the description which follows and, in part, will be apparent from the description, or may be learned by practice of the presented embodiments.

The present invention refers to a heterocyclic compound selected from Compounds 2, 5, 9, 25, 31, 40, 45, 60, 74, 81, and 90:

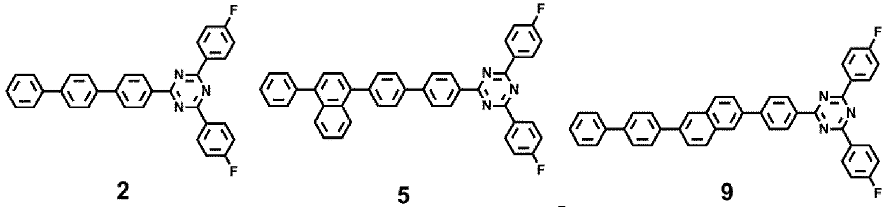

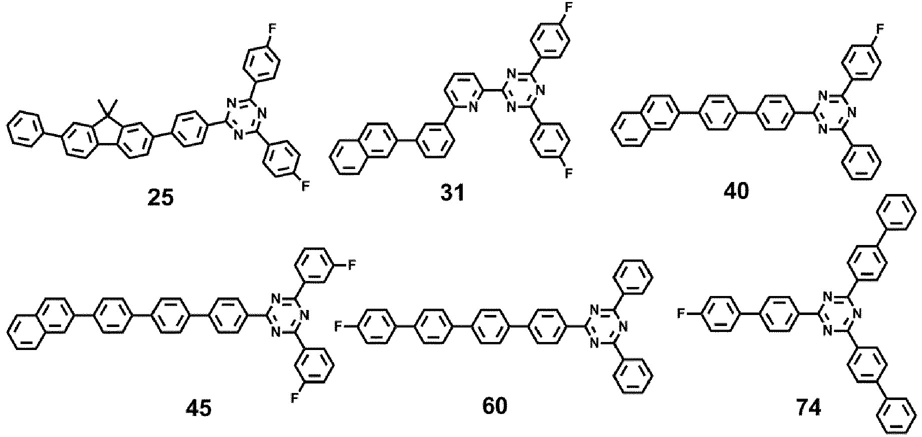

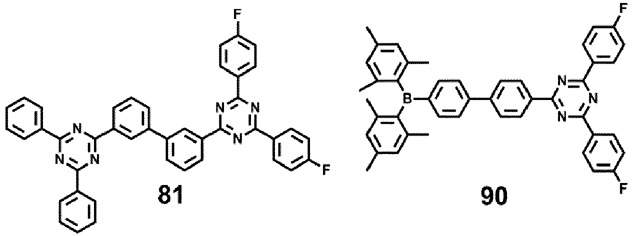

Another aspect of an embodiment of the present disclosure provides an organic light-emitting device including: a first electrode; a second electrode facing the first electrode; and an organic layer between the first electrode and the second electrode and including an organic layer, wherein the organic layer includes an emission layer and at least one of the heterocyclic compound.

### BRIEF DESCRIPTION OF THE DRAWINGS

These and/or other aspects of embodiments will become apparent and more readily appreciated from the following description of the embodiments, taken in conjunction with the accompanying drawings in which:
FIG. 1 is a schematic view of an organic light-emitting device according to an embodiment;
FIG. 2 is a schematic view of an organic light-emitting device according to an embodiment;
FIG. 3 is a schematic view of an organic light-emitting device according to an embodiment; and
FIG. 4 is a schematic view of an organic light-emitting device according to an embodiment.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the drawings, to explain aspects of embodiments of the present description. As used herein, the term "and/or" includes any and all combinations of one or more of the associated listed items. Expressions such as "at least one of," when preceding a list of elements, modify the entire list of elements and do not modify the individual elements of the list.

The heterocyclic compound selected from Compounds 2, 5, 9, 25, 31, 40, 45, 60, 74, 81, and 90:

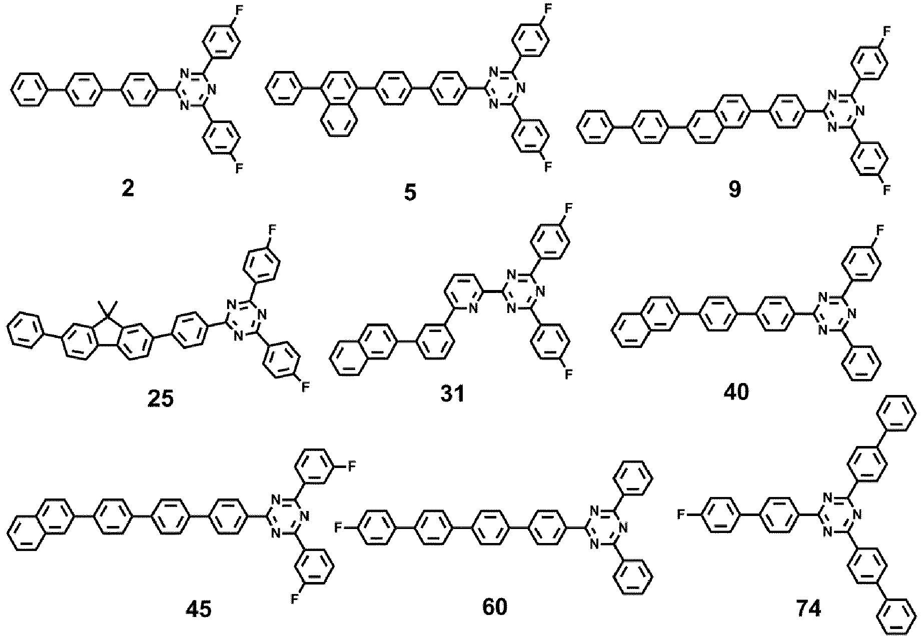

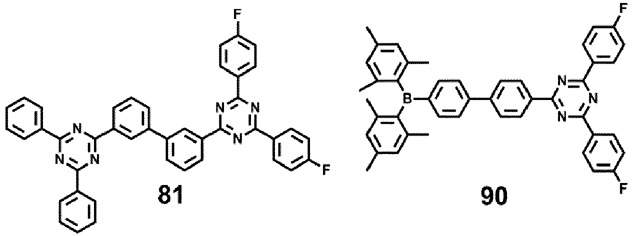

While the present application is not limited by any particular mechanism or theory it showed up, that since the heterocyclic compound includes at least one fluoro (fluorine) that is an element having high electronegativity, heterocyclic compound molecules form a strong dipole (e.g., each molecule of the heterocyclic compound may have a strong dipole moment). It further showed, without limiting the present application, that as a result, an interfacial vacuum level is lowered by intermolecular interfacial dipole-dipole interaction (e.g., intermolecular interfacial dipole-dipole interaction between molecules of the heterocyclic compound), thereby facilitating electron injection. Also, it turned out to be that even in an electron transport layer (ETL), electron transport is facilitated by strong secondary bonding such as hydrogen bonding caused by unshared electrons of the fluoro element (fluorine), but the present application is not limited by any particular mechanism or theory.

Also, since the heterocyclic compound does not include another triazine group between a terminus including the fluoro-containing cyclic group and a triazine core, a molecular structure of the heterocyclic compound may be designed to be not symmetrical but asymmetrical. Accordingly, the molecules may have stronger polarity.

A synthesis method for the above-mentioned heterocyclic compounds would be apparent to those of ordinary skill in the art by referring to the following examples.

At least one of the above-mentioned heterocyclic compounds may be used between a pair of electrodes of an organic light-emitting device. For example, the heterocyclic compound may be included in at least one layer selected from a hole transport region, an electron transport region, and an emission layer. In one or more embodiments, the above-mentioned heterocyclic compounds may be used as a material for a capping layer located outside a pair of electrodes of an organic light-emitting device.

Accordingly, provided is an organic light-emitting device including: a first electrode; a second electrode facing the first electrode; and an organic layer between the first electrode and the second electrode and including an emission layer, wherein the organic layer includes at least one condensed cyclic compound.

The expression "(an organic layer) includes at least one heterocyclic compound," as used herein, may include a case in which "(an organic layer) includes identical compounds selected from Compounds 2, 5, 9, 25, 31, 40, 45, 60, 74, 81, and 90" and a case in which "(an organic layer) includes two or more different heterocyclic compounds."

In one embodiment, the first electrode is an anode, and the second electrode is a cathode. Preferably, the organic layer further comprises a hole transport region between the first electrode, an emission layer and/or an electron transport region which is preferably disposed between the emission layer and the second electrode. Optionally, the hole transport region includes a hole injection layer, a hole transport layer, an emission auxiliary layer, an electron blocking layer, and/or any combination thereof, and the electron transport region includes a buffer layer, a hole blocking layer, an emission auxiliary layer, an electron control layer, an electron transport layer, an electron injection layer, and/or any combination thereof.

In one embodiment, the electron transport region may include at least one of the heterocyclic compounds disclosed herein.

In one or more embodiments, the electron transport region may include an electron transport layer, and the electron transport layer may include the heterocyclic compound.

In one or more embodiments, the electron transport region may include an emission auxiliary layer, and the emission auxiliary layer may include the heterocyclic compound.

In one or more embodiments, the hole transport region may include a p-dopant, and the p-dopant may have a lowest unoccupied molecular orbital (LUMO) energy level of not more than -3 eV, more preferred not more than -3.25 eV, prefferably -3.5 eV or less.

In one or more embodiments, the p-dopant may include a cyano group-containing compound.

The host in the emission layer may include at least one selected from an anthracene-based compound, a pyrene-based compound, and a spiro-bifluorene-based compound, but embodiments of the present disclosure are not limited thereto.

The dopant in the emission layer may include at least one selected from a styryl-based compound and an amine-based compound, but embodiments of the present disclosure are not limited thereto.

In the organic light-emitting device, the emission layer may be a first emission layer for emitting first color light,
between the first electrode and second electrode, i) at least one second emission layer for emitting second color light may be further included, and ii) at least one second emission layer for emitting second color light and/or at least one third emission layer for emitting third color light may be further included,
a maximum (e.g., an upper end) emission wavelength of the first color light, a maximum (e.g., an upper end) emission wavelength of the second color light, and/or a maximum (e.g., an upper end) emission wavelength of the third color light may be identical to or different from each other, additionally or alternatively
the first color light and the second color light may be emitted in the form of mixed light, or the first color light, the second color light, and the third color light may be emitted in the form of mixed light.

The organic light-emitting device may further include at least one selected from a first capping layer disposed in a pathway along which light generated in an emission layer proceeds toward the outside through the first electrode and a second capping layer disposed in a pathway along which light generated in an emission layer proceeds toward the outside through the second electrode, and the at least one selected from the first capping layer and the second capping layer may include at least one of the above-mentioned heterocyclic compounds.

For example, the organic light-emitting device may have i) a stacked structure including a first electrode, an organic layer, a second electrode, and a second capping layer which are sequentially stacked in this stated order, ii) a stacked structure including a first capping layer, a first electrode, an organic layer, and a second electrode which are sequentially stacked in this stated order, or iii) a stacked structure including a first capping layer, a first electrode, an organic layer, a second electrode, and a second capping layer which are sequentially stacked in this stated order, and at least one selected from the first capping layer and the second capping layer may include the heterocyclic compound.

The term "organic layer," as used herein, refers to a single layer and/or a plurality of layers disposed between the first electrode and the second electrode of the organic light-emitting device. A material included in the "organic layer" is not limited to an organic material.

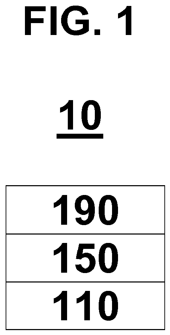

### Description of FIG. 1

FIG. 1 is a schematic view of an organic light-emitting device 10 according to an embodiment. The organic light-emitting device 10 includes a first electrode 110, an organic layer 150, and a second electrode 190.

Hereinafter, the structure of the organic light-emitting device 10 according to an embodiment and a method of manufacturing the organic light-emitting device 10 will be described in connection with FIG. 1.

### [First electrode 110]

In FIG. 1, a substrate may be additionally disposed under the first electrode 110 or above the second electrode 190. The substrate may be a glass substrate or a plastic substrate, each having excellent mechanical strength, thermal stability, transparency, surface smoothness, ease of handling, and water resistance.

The first electrode 110 may be formed by depositing or sputtering a material for forming the first electrode 110 on the substrate. When the first electrode 110 is an anode, the material for a first electrode may be selected from materials having a high work function to facilitate hole injection.

The first electrode 110 may be a reflective electrode, a semi-transmissive electrode, or a transmissive electrode. When the first electrode 110 is a transmissive electrode, a material for forming a first electrode may be selected from indium tin oxide (ITO), indium zinc oxide (IZO), tin oxide (SnO₂), zinc oxide (ZnO), and any combinations thereof, but embodiments of the present disclosure are not limited thereto. In one or more embodiments, when the first electrode 110 is a semi-transmissive electrode or a reflectable (reflective) electrode, a material for forming a first electrode may be selected from magnesium (Mg), silver (Ag), aluminum (Al), aluminum-lithium (Al-Li), calcium (Ca), magnesium-indium (Mg-In), magnesium-silver (Mg-Ag), and any combinations thereof, but embodiments of the present disclosure are not limited thereto.

The first electrode 110 may have a single-layered structure, or a multi-layered structure including two or more layers. For example, the first electrode 110 may have a three-layered structure of ITO/Ag/ITO, but the structure of the first electrode 110 is not limited thereto.

### [Organic layer 150]

The organic layer 150 is disposed on the first electrode 110. The organic layer 150 includes a compound selected from Compounds 2, 5, 9, 25, 31, 40, 45, 60, 74, 81, and 90 and may include an emission layer.

The organic layer 150 may further include a hole transport region between the first electrode 110 and the emission layer, and an electron transport region between the emission layer and the second electrode 190.

### [Hole transport region in organic layer 150]

The hole transport region may have i) a single-layered structure including a single layer including a single material, ii) a single-layered structure including a single layer including a plurality of different materials, or iii) a multi-layered structure having a plurality of layers including a plurality of different materials.

The hole transport region may include at least one layer selected from a hole injection layer, a hole transport layer, an emission auxiliary layer, and an electron blocking layer.

For example, the hole transport region may have a single-layered structure including a single layer including a plurality of different materials, or a multi-layered structure having a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron blocking layer structure, wherein for each structure, constituting layers are sequentially stacked from the first electrode 110 in this stated order, but the structure of the hole transport region is not limited thereto.

The hole transport region may include at least one selected from m-MTDATA, TDATA, 2-TNATA, NPB(NPD), β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201, and a compound represented by Formula 202:

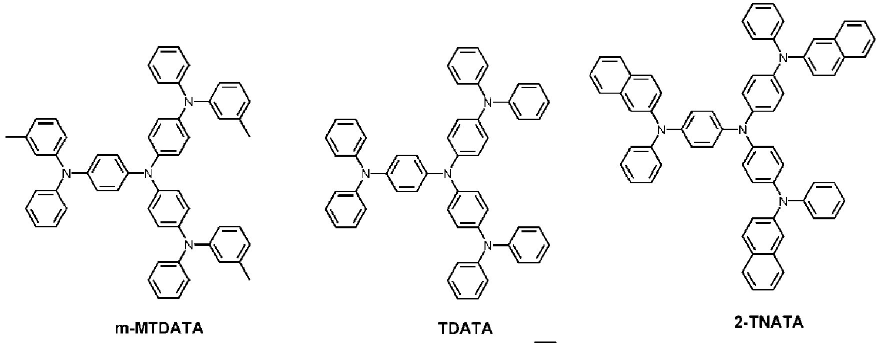

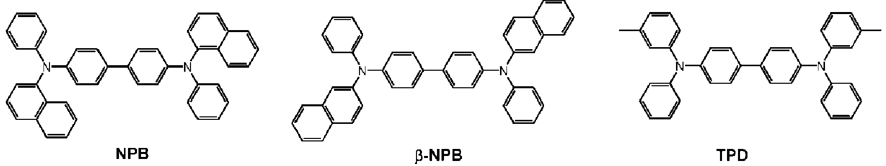

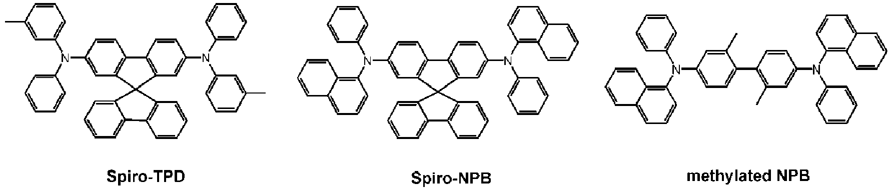

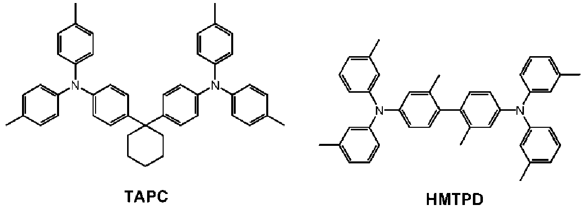

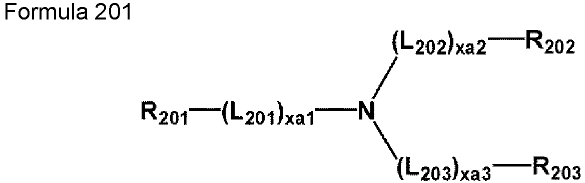

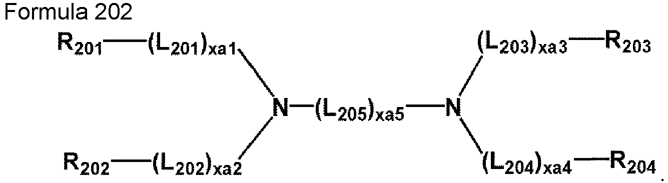

In Formulae 201 and 202,
L₂₀₁ to L₂₀₄ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
L₂₀₅ may be selected from *-O-*', *-S-*', *-N(Q₂₀₁)-*', a substituted or unsubstituted C₁-C₂₀ alkylene group, a substituted or unsubstituted C₂-C₂₀ alkenylene group, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xa1 to xa4 may each independently be an integer from 0 to 3,
xa5 may be an integer from 1 to 10, and
R₂₀₁ to R₂₀₄ and Q₂₀₁ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group.

For example, in Formula 202, R₂₀₁ and R₂₀₂ may optionally be linked via a single bond, a dimethyl-methylene group, or a diphenyl-methylene group, and R₂₀₃ and R₂₀₄ may optionally be linked via a single bond, a dimethyl-methylene group, or a diphenyl-methylene group.

In one embodiment, in Formulae 201 and 202,
L₂₀₁ to L₂₀₅ may each independently be selected from:
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In one or more embodiments, xa1 to xa4 may each independently be 0, 1, or 2.

In one or more embodiments, xa5 may be 1, 2, 3, or 4.

In one or more embodiments, R₂₀₁ to R₂₀₄ and Q₂₀₁ may each independently be selected from:
a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂), and
Q₃₁ to Q₃₃ are the same as described elsewhere herein.

In one or more embodiments, at least one selected from R₂₀₁ to R₂₀₃ in Formula 201 may independently be selected from:
a fluorenyl group, a spiro-bifluorenyl group, a carbazolyl group, a dibenzofuranyl group, and a dibenzothiophenyl group; and
a fluorenyl group, a spiro-bifluorenyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group,
but embodiments of the present disclosure are not limited thereto.

In one or more embodiments, in Formula 202, i) R₂₀₁ and R₂₀₂ may be linked via a single bond, and/or ii) R₂₀₃ and R₂₀₄ may be linked via a single bond.

In one or more embodiments, at least one selected from R₂₀₁ to R₂₀₄ in Formula 202 may each independently be selected from:
a carbazolyl group; and
a carbazolyl group substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group,
but embodiments of the present disclosure are not limited thereto.

The compound represented by Formula 201 may be represented by Formula 201A:

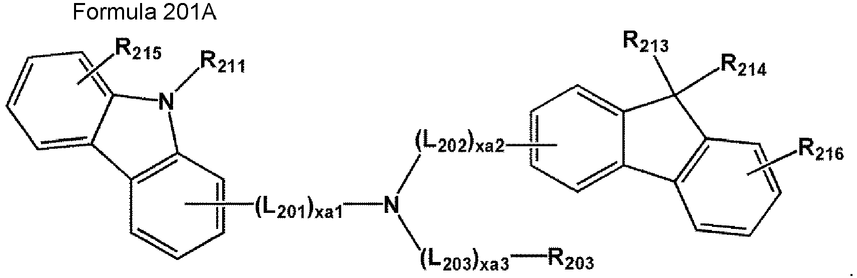

In one embodiment, the compound represented by Formula 201 may be represented by Formula 201A(1) below, but embodiments of the present disclosure are not limited thereto:

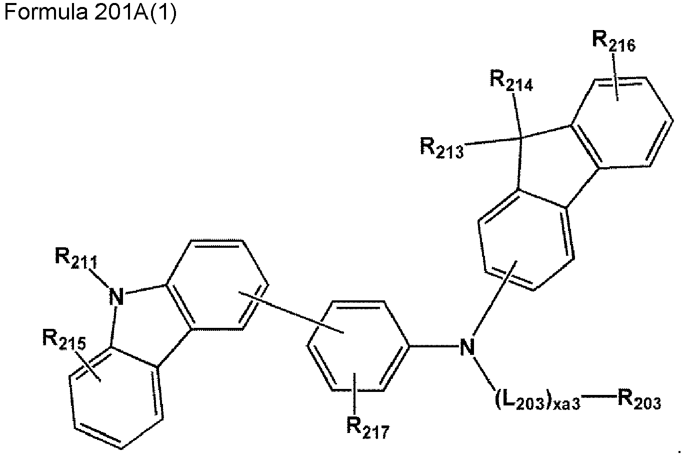

In one embodiment, the compound represented by Formula 201 may be represented by Formula 201A-1 below, but embodiments of the present disclosure are not limited thereto:

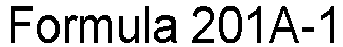

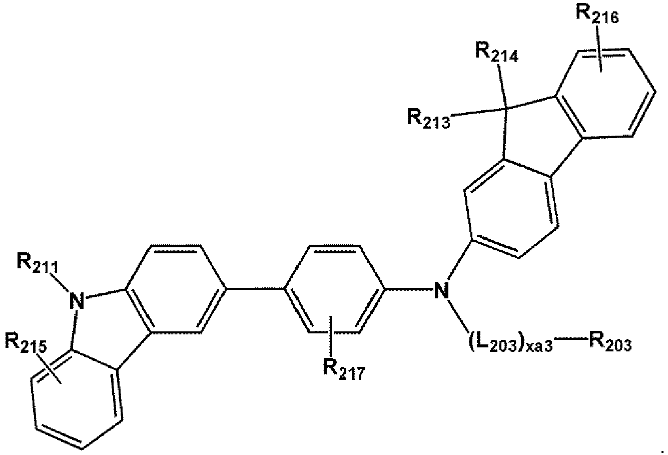

In one embodiment, the compound represented by Formula 202 may be represented by Formula 202A:

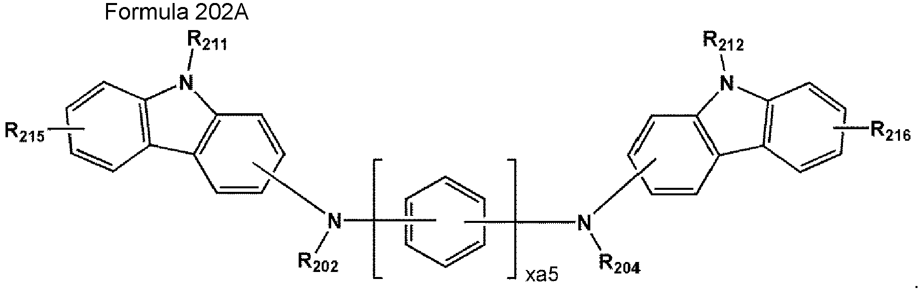

In one embodiment, the compound represented by Formula 202 may be represented by Formula 202A-1:

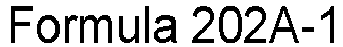

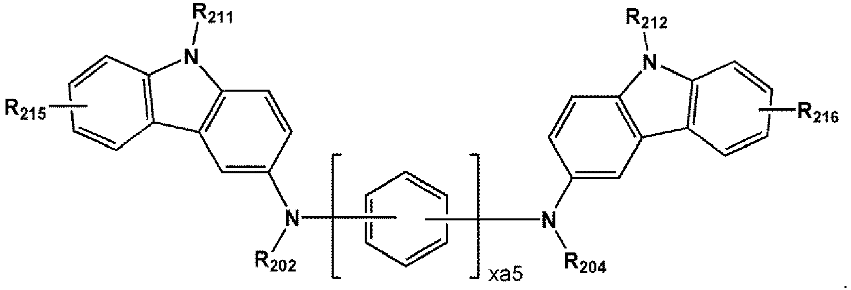

In Formulae 201A, 201A(1), 201A-1, 202A, and 202A-1,
L₂₀₁ to L₂₀₃, xa1 to xa3, xa5, and R₂₀₂ to R₂₀₄ are the same as described above,
R₂₁₁ and R₂₁₂ may be understood by referring to the description provided herein in connection with R₂₀₃, and
R₂₁₃ to R₂₁₇ may each independently be selected from hydrogen, deuterium, -F,-Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

The hole transport region may include at least one compound selected from Compounds HT1 to HT39, but embodiments of the present disclosure are not limited thereto:

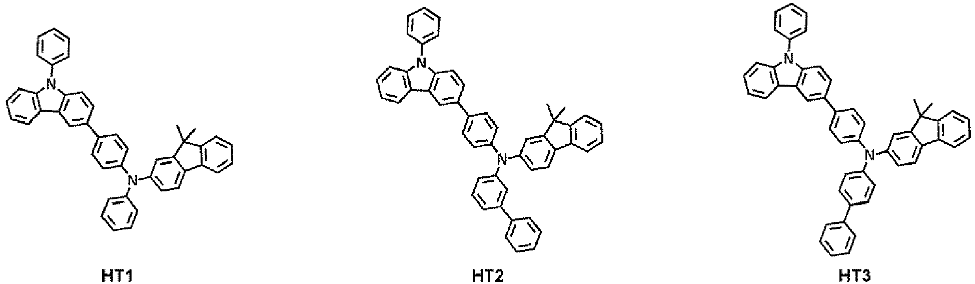

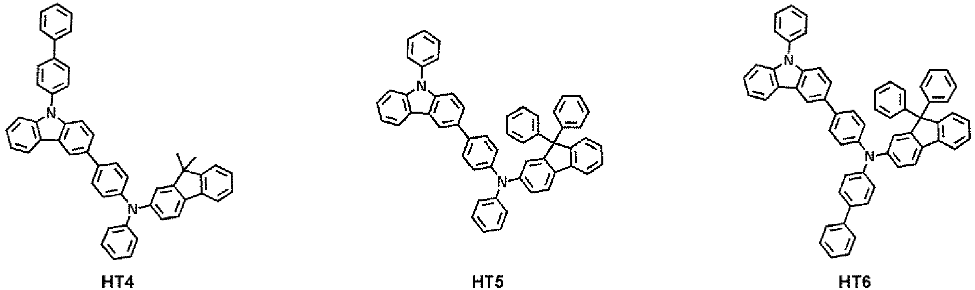

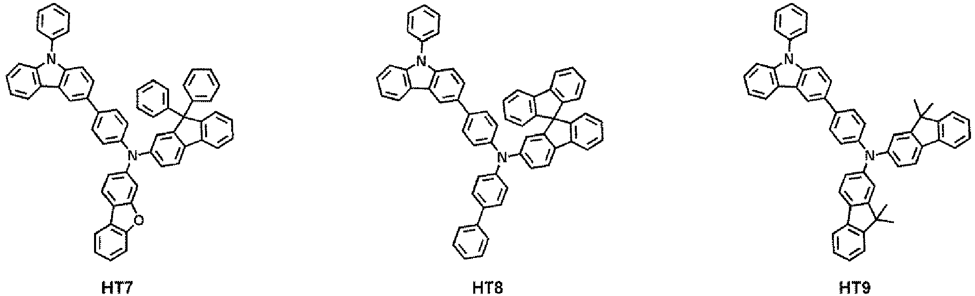

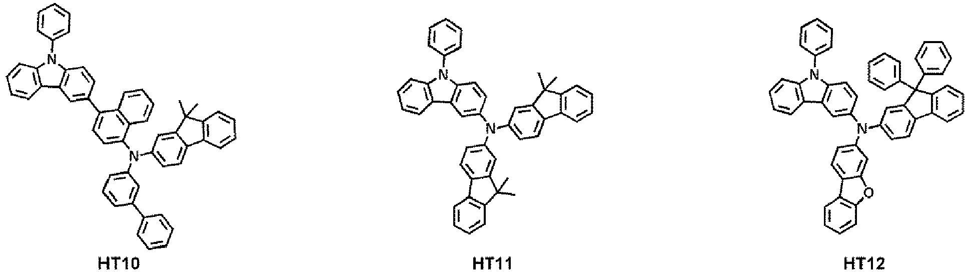

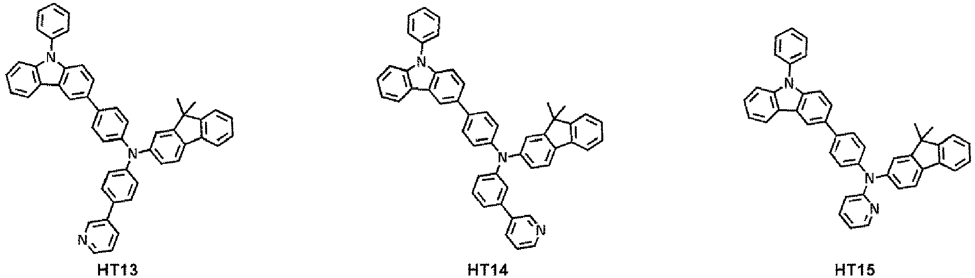

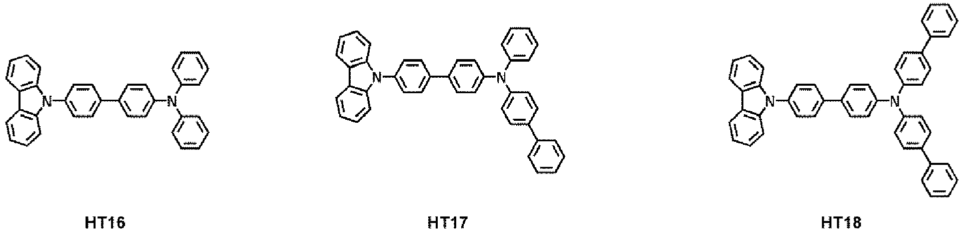

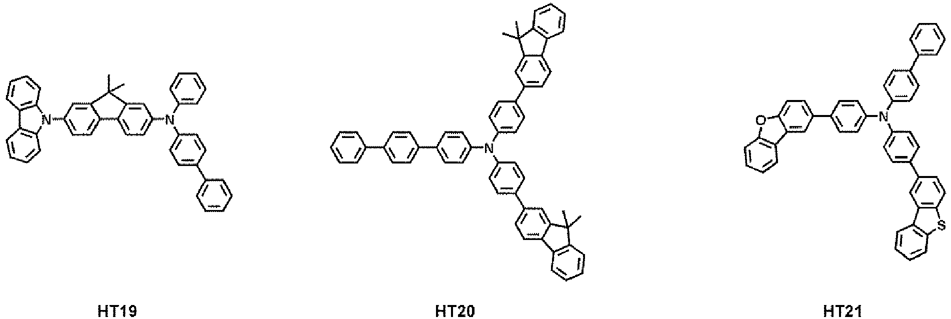

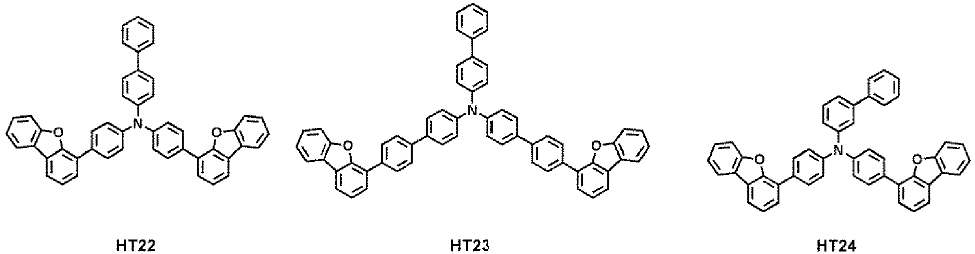

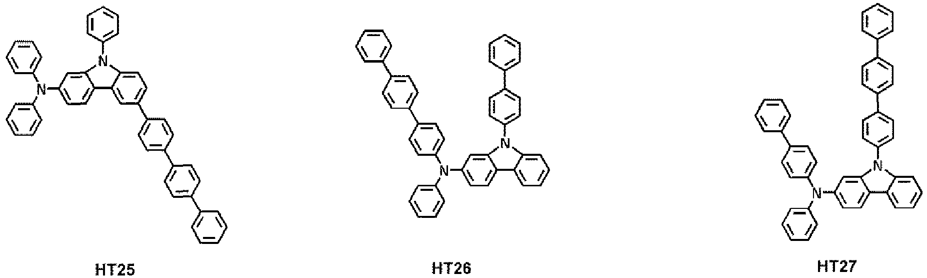

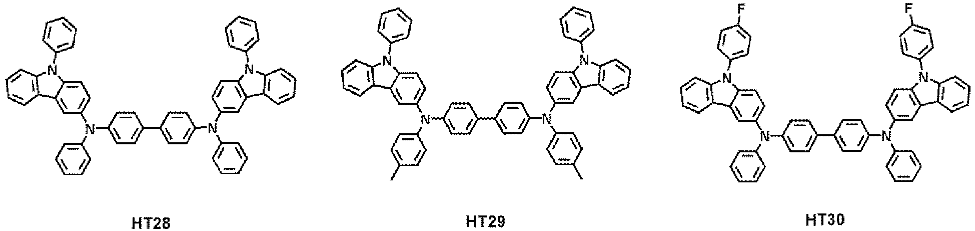

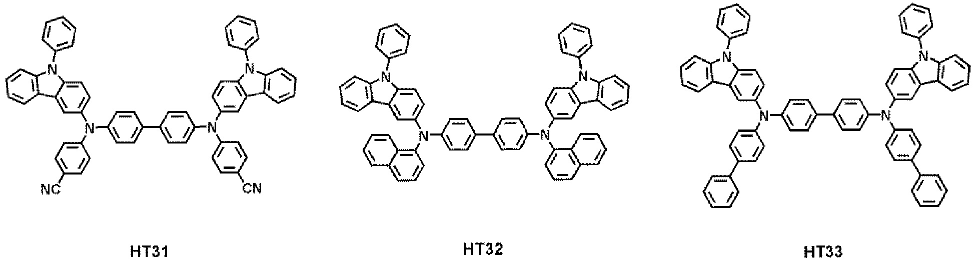

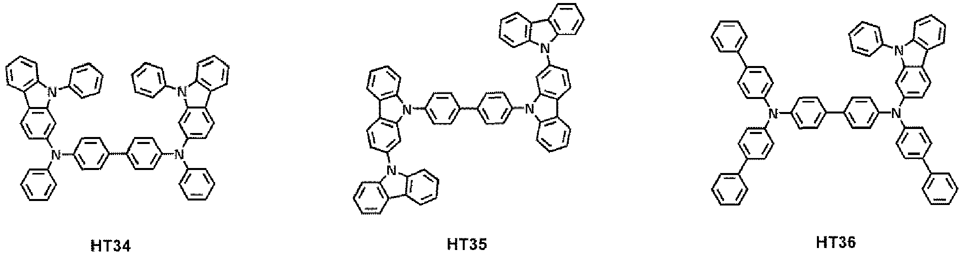

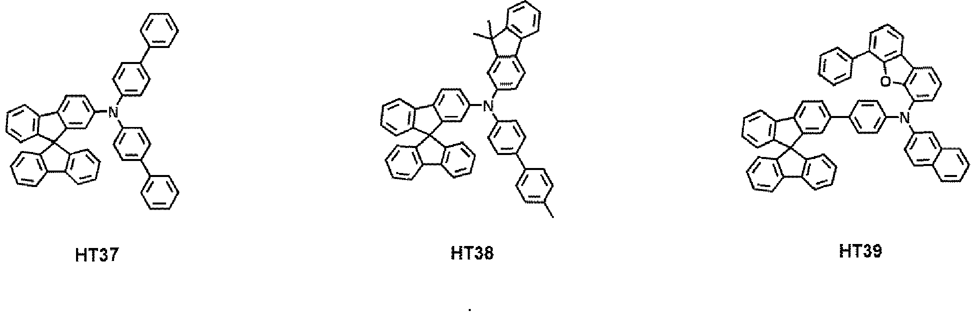

A thickness of the hole transport region may be in a range of about 100 Å to about 10,000 Å, for example, about 100 Å to about 1,000 Å. When the hole transport region includes at least one selected from a hole injection layer and a hole transport layer, the thickness of the hole injection layer may be in a range of about 100 Å to about 9,000 Å, and for example, about 100 Å to about 1,000 Å, and the thickness of the hole transport layer may be in a range of about 50 Å to about 2,000 Å, and for example, about 100 Å to about 1500 Å. When the thicknesses of the hole transport region, the hole injection layer, and the hole transport layer are within these ranges, suitable or satisfactory hole transporting characteristics may be obtained without a substantial increase in driving voltage.

The emission auxiliary layer may increase light-emission efficiency by compensating for an optical resonance distance according to the wavelength of light emitted by an emission layer, and the electron blocking layer may block the flow of electrons from an electron transport region. The emission auxiliary layer and the electron blocking layer may include the materials as described above.

### [p-dopant]

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant.

In one embodiment, the p-dopant may have a LUMO of about -3.5 eV or less.

The p-dopant may include at least one selected from a quinone derivative, a metal oxide, and a cyano group-containing compound, but embodiments of the present disclosure are not limited thereto.

For example, the p-dopant may include at least one selected from:
a quinone derivative, such as tetracyanoquinodimethane (TCNQ) or 2,3,5,6-tetrafluoro-7,7,8,8-tetracyanoquinodimethane (F4-TCNQ);
a metal oxide, such as tungsten oxide or molybdenum oxide;
1,4,5,8,9,11-hexaazatriphenylene-hexacarbonitrile (HAT-CN); and
a compound represented by Formula 221 below:
but embodiments of the present disclosure are not limited thereto:

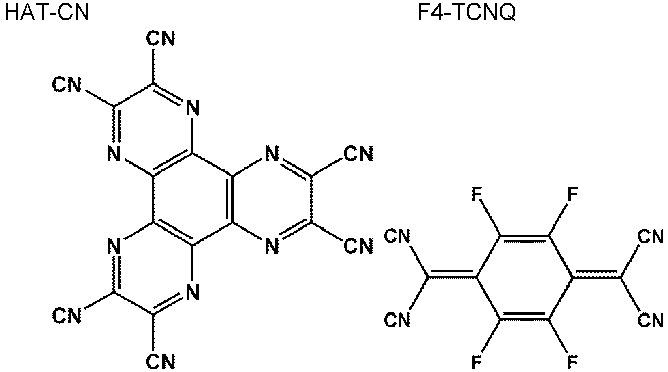

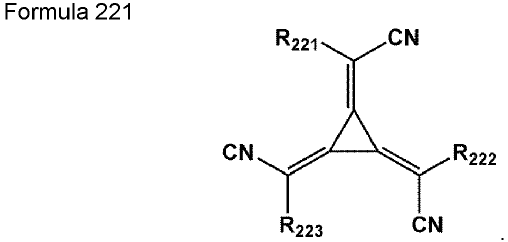

In Formula 221,
R₂₂₁ to R₂₂₃ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, provided that at least one selected from R₂₂₁ to R₂₂₃ has at least one substituent selected from a cyano group, -F, -Cl, -Br, -I, a C₁-C₂₀ alkyl group substituted with -F, a C₁-C₂₀ alkyl group substituted with -Cl, a C₁-C₂₀ alkyl group substituted with -Br, and a C₁-C₂₀ alkyl group substituted with -I.

### [Emission layer in organic layer 150]

When the organic light-emitting device 10 is a full-color organic light-emitting device, the emission layer may be patterned into a red emission layer, a green emission layer, or a blue emission layer, according to a sub-pixel. In one or more embodiments, the emission layer may have a stacked structure of two or more layers selected from a red emission layer, a green emission layer, and a blue emission layer, in which the two or more layers contact each other or are separated from each other. In one or more embodiments, the emission layer may include two or more materials selected from a red light-emitting material, a green light-emitting material, and a blue light-emitting material, in which the two or more materials are mixed with each other in a single layer to emit white light.

The emission layer may include a host and a dopant. The dopant may include at least one selected from a phosphorescent dopant and a fluorescent dopant.

An amount of the dopant in the emission layer may be in a range of about 0.01 parts by weight to about 15 parts by weight based on 100 parts by weight of the host, but embodiments of the present disclosure are not limited thereto.

A thickness of the emission layer may be in a range of about 100 Å to about 1,000 Å, for example, about 200 Å to about 600 Å. When the thickness of the emission layer is within this range, excellent light-emission characteristics may be obtained without a substantial increase in driving voltage.

### [Host in emission layer]

In one or more embodiments, the host may further include a compound represented by Formula 301 below:

Formula 301 [Ar₃₀₁]_{xb11}-[(L₃₀₁)_{xb1}-R₃₀₁]_{xb21}.

In Formula 301,
Ar₃₀₁ may be a substituted or unsubstituted C₅-C₆₀ carbocyclic group or a substituted or unsubstituted C₁-C₆₀ heterocyclic group,
xb11 may be 1, 2, or 3,
L₃₀₁ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group;
xb1 may be an integer from 0 to 5,
R₃₀₁ may be selected from deuterium, -F, -Cl, -Br, -I, a hydroxyl group, a cyano group, a nitro group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₃₀₁)(Q₃₀₂)(Q₃₀₃), -N(Q₃₀₁)(Q₃₀₂), -B(Q₃₀₁)(Q₃₀₂),-C(=O)(Q₃₀₁), -S(=O)₂(Q₃₀₁), and -P(=O)(Q₃₀₁)(Q₃₀₂),
xb21 may be an integer from 1 to 5, and
Q₃₀₁ to Q₃₀₃ may each independently be selected from a C₁-C₁₀ alkyl group, a C₁-C₁₀ alkoxy group, a phenyl group, a biphenyl group, a terphenyl group, and a naphthyl group, but embodiments of the present disclosure are not limited thereto.

In one embodiment, in Formula 301, Ar₃₀₁ may be selected from:
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group; and
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -N(Q₃₁)(Q₃₂), -B(Q₃₁)(Q₃₂), -C(=O)(Q₃₁),-S(=O)₂(Q₃₁), and -P(=O)(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group, but embodiments of the present disclosure are not limited thereto.

In Formula 301, xb11 may be two or more, and two or more of groups Ar₃₀₁ may be linked via a single bond.

In one or more embodiments, the compound represented by Formula 301 may be represented by Formula 301-1 or 301-2:

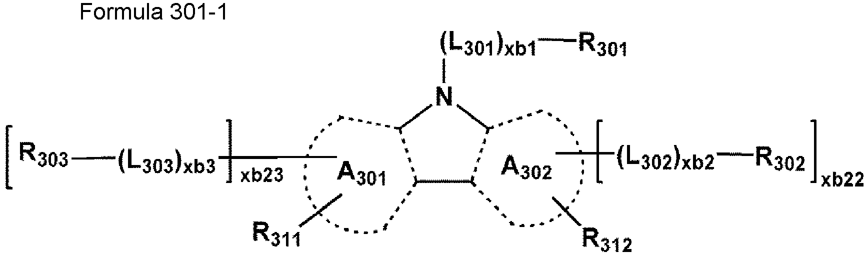

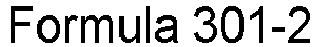

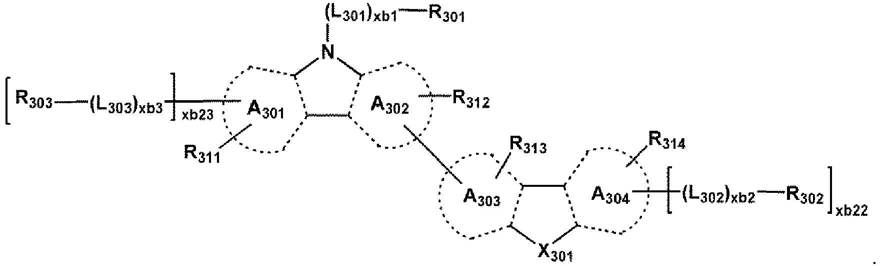

In Formulae 301-1 and 301-2,
A₃₀₁ to A₃₀₄ may each independently be selected from a benzene group, a naphthalene group, a phenanthrene group, a fluoranthene group, a triphenylene group, a pyrene group, a chrysene group, a pyridine group, a pyrimidine group, an indene group, a fluorene group, a spiro-bifluorene group, a benzofluorene group, a dibenzofluorene group, an indole group, a carbazole group, a benzocarbazole group, a dibenzocarbazole group, a furan group, a benzofuran group, a dibenzofuran group, a naphthofuran group, a benzonaphthofuran group, a dinaphthofuran group, a thiophene group, a benzothiophene group, a dibenzothiophene group, a naphthothiophene group, a benzonaphthothiophene group, and a dinaphthothiophene group,
X₃₀₁ may be O, S, or N-[(L₃₀₄)_{xb4}-R₃₀₄],
R₃₁₁ to R₃₁₄ may each independently be selected from hydrogen, deuterium, -F,-Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group -Si(Q₃₁)(Q₃₂)(Q₃₃), -N(Q₃₁)(Q₃₂),-B(Q₃₁)(Q₃₂), -C(=O)(Q₃₁), -S(=O)₂(Q₃₁) and -P(=O)(Q₃₁)(Q₃₂),
xb22 and xb23 may each independently be 0, 1, or 2,
L₃₀₁, xb1, R₃₀₁, and Q₃₁ to Q₃₃ may each independently be the same as described above,
L₃₀₂ to L₃₀₄ may each independently be the same as described in connection with L₃₀₁,
xb2 to xb4 may each independently be the same as described in connection with xb1, and
R₃₀₂ to R₃₀₄ may each independently be the same as described in connection with R₃₀₁.

For example, in Formulae 301, 301-1, and 301-2, L₃₀₁ to L₃₀₄ may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F,-Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂),-B(Q₃₁)(Q₃₂), -C(=O)(Q₃₁), -S(=O)₂(Q₃₁), and -P(=O)(Q₃₁)(Q₃₂), and
Q₃₁ to Q₃₃ may be the same as described above.

In one embodiment, in Formulae 301, 301-1, and 301-2, R₃₀₁ to R₃₀₄ may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), -B(Q₃₁)(Q₃₂), -C(=O)(Q₃₁),-S(=O)₂(Q₃₁), and -P(=O)(Q₃₁)(Q₃₂), and
Q₃₁ to Q₃₃ may be the same as described above.

In one or more embodiments, the host may include an alkaline earth metal complex. For example, the host may be selected from a Be complex (for example, Compound H55), a Mg complex, and a Zn complex.

The host may include at least one selected from 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), and Compounds H1 to H55, but embodiments of the present disclosure are not limited thereto:

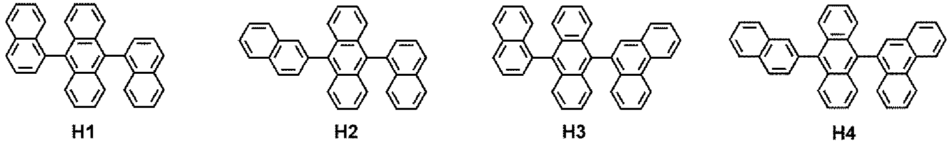

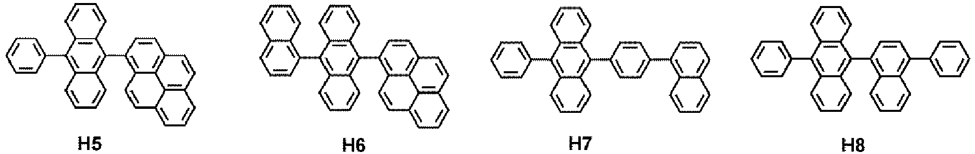

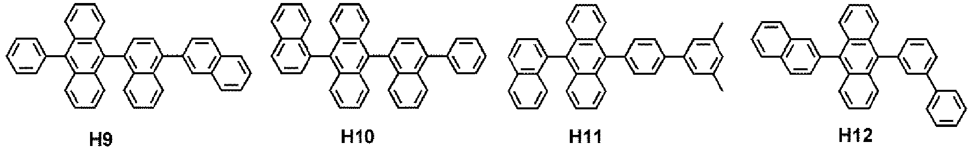

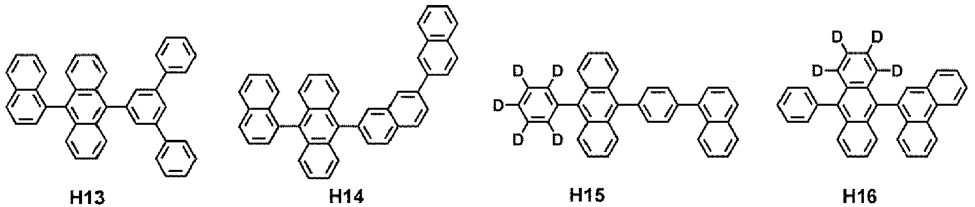

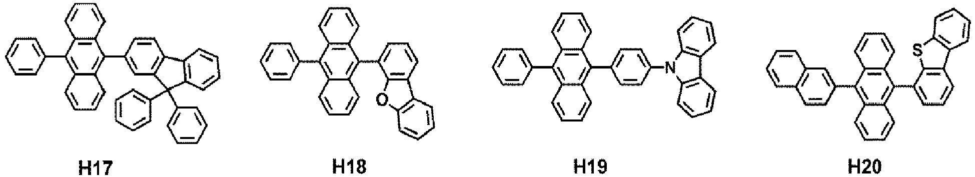

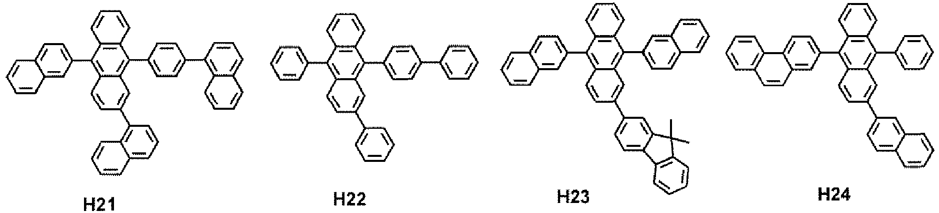

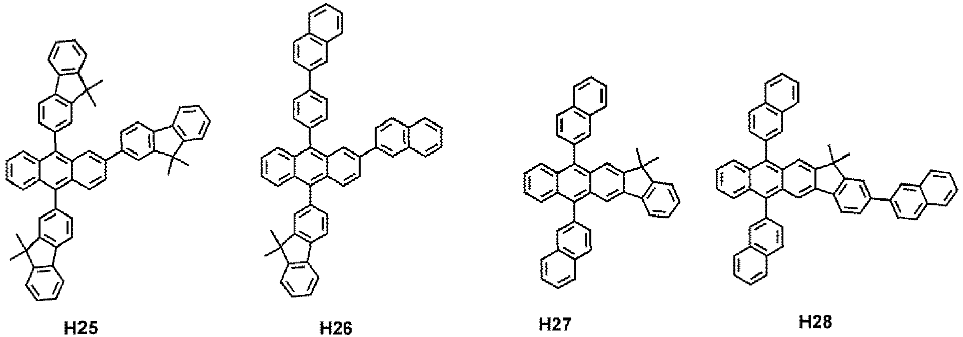

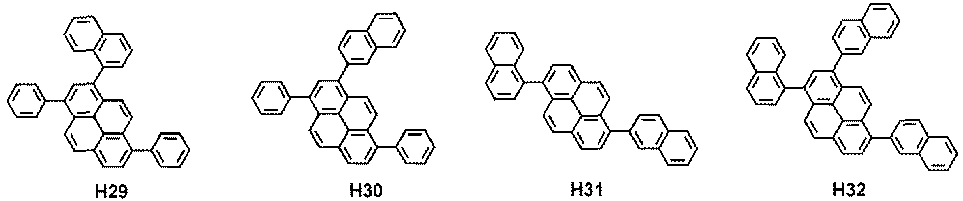

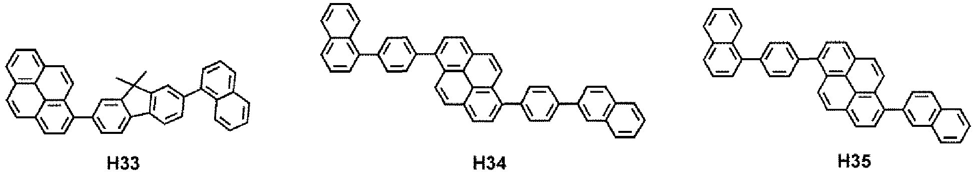

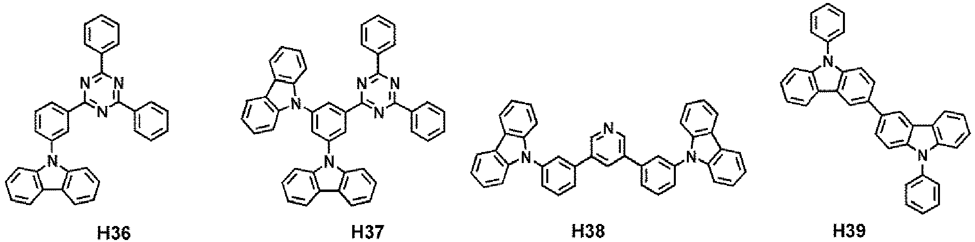

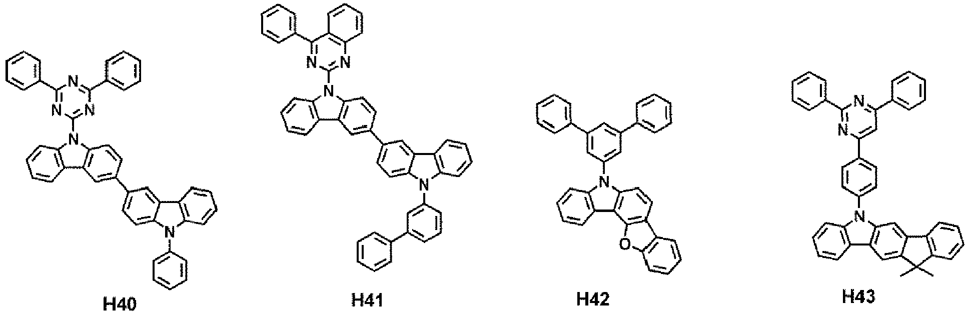

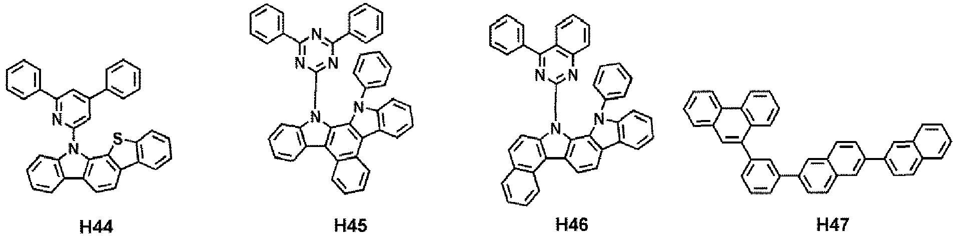

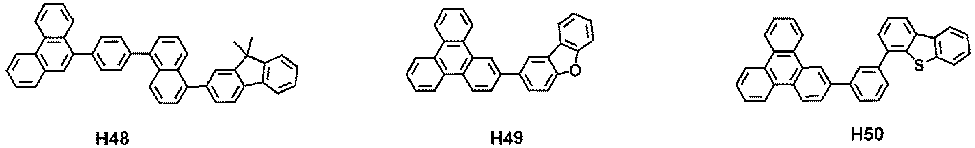

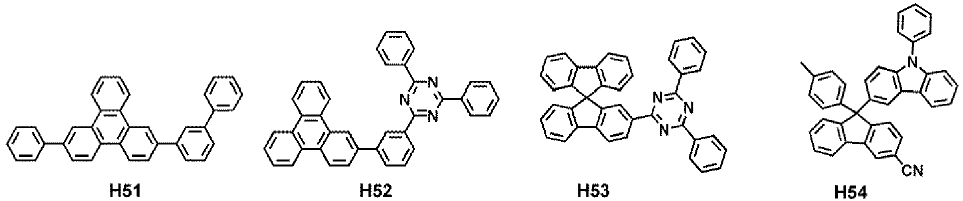

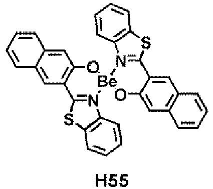

### [Phosphorescent dopant included in emission layer in organic layer 150]

The phosphorescent dopant may include an organometallic complex represented by Formula 401 below:

Formula 401 M(L₄₀₁)_{xc1}(L₄₀₂)_{xc2}

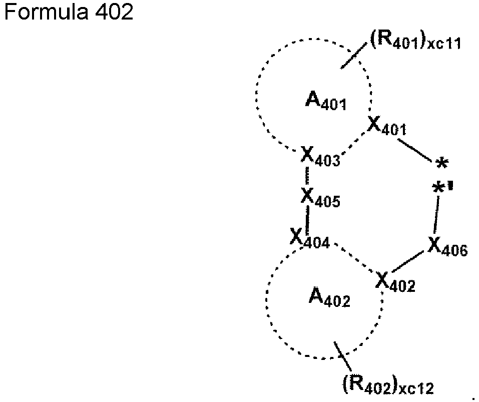

In Formulae 401 and 402,
M may be selected from iridium (Ir), platinum (Pt), palladium (Pd), osmium (Os), titanium (Ti), zirconium (Zr), hafnium (Hf), europium (Eu), terbium (Tb), rhodium (Rh), and thulium (Tm),
L₄₀₁ may be selected from ligands represented by Formula 402, and xc1 may be 1, 2, or 3, wherein, when xc1 is two or more, two or more L₄₀₁(s) may be identical to or different from each other,
L₄₀₂ may be an organic ligand, and xc2 may be an integer from 0 to 4, wherein, when xc2 is two or more, two or more L₄₀₂(s) may be identical to or different from each other,
X₄₀₁ to X₄₀₄ may each independently be nitrogen or carbon,
X₄₀₁ and X₄₀₃ may be linked via a single bond or a double bond, and X₄₀₂ and X₄₀₄ may be linked via a single bond or a double bond,
A₄₀₁ and A₄₀₂ may each independently be selected from a C₅-C₆₀ carbocyclic group or a C₁-C₆₀ heterocyclic group,
X₄₀₅ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C=*', and Q₄₁₁ and Q₄₁₂ may each independently be hydrogen, deuterium, a C₁-C₂₀ alkyl group, a C₁-C₂₀ alkoxy group, a phenyl group, a biphenyl group, a terphenyl group, or a naphthyl group,
X₄₀₆ may be a single bond, O, or S,
R₄₀₁ and R₄₀₂ may each independently be selected from hydrogen, deuterium,-F, -Cl, -Br, -I, a hydroxyl group, a cyano group, a nitro group, an amidino group, a hydrazino group, a hydrazono group, a substituted or unsubstituted C₁-C₂₀ alkyl group, a substituted or unsubstituted C₁-C₂₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₄₀₁)(Q₄₀₂)(Q₄₀₃),-N(Q₄₀₁)(Q₄₀₂), -B(Q₄₀₁)(Q₄₀₂), -C(=O)(Q₄₀₁), -S(=O)₂(Q₄₀₁), and -P(=O)(Q₄₀₁)(Q₄₀₂),
Q₄₀₁ to Q₄₀₃ may each independently be selected from a C₁-C₁₀ alkyl group, a C₁-C₁₀ alkoxy group, a C₆-C₂₀ aryl group, and a C₁-C₂₀ heteroaryl group,
xc11 and xc12 may each independently be an integer from 0 to 10, and
* and *' in Formula 402 each indicate a binding site to M in Formula 401.

In one embodiment, A₄₀₁ and A₄₀₂ in Formula 402 may each independently be selected from a benzene group, a naphthalene group, a fluorene group, a spiro-bifluorene group, an indene group, a pyrrole group, a thiophene group, a furan group, an imidazole group, a pyrazole group, a thiazole group, an isothiazole group, an oxazole group, an isoxazole group, a pyridine group, a pyrazine group, a pyrimidine group, a pyridazine group, a quinoline group, an isoquinoline group, a benzoquinoline group, a quinoxaline group, a quinazoline group, a carbazole group, a benzimidazole group, a benzofuran group, a benzothiophene group, an isobenzothiophene group, a benzoxazole group, an isobenzoxazole group, a triazole group, a tetrazole group, an oxadiazole group, a triazine group, a dibenzofuran group, and a dibenzothiophene group.

In one or more embodiments, in Formula 402, i) X₄₀₁ may be nitrogen, and X₄₀₂ may be carbon, or ii) X₄₀₁ and X₄₀₂ may each be nitrogen at the same time.

In one or more embodiments, R₄₀₂ and R₄₀₂ in Formula 401 may each independently be selected from:
hydrogen, deuterium, -F, -Cl, -Br, -I, a hydroxyl group, a cyano group, a nitro group, an amidino group, a hydrazino group, a hydrazono group, a C₁-C₂₀ alkyl group, and a C₁-C₂₀ alkoxy group;
a C₁-C₂₀ alkyl group and a C₁-C₂₀ alkoxy group, each substituted with at least one selected from deuterium, -F, -Cl, -Br, -I, a hydroxyl group, a cyano group, a nitro group, an amidino group, a hydrazino group, a hydrazono group, a phenyl group, a naphthyl group, a cyclopentyl group, a cyclohexyl group, an adamantanyl group, a norbornanyl group, and a norbornenyl group;
a cyclopentyl group, a cyclohexyl group, an adamantanyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group;
a cyclopentyl group, a cyclohexyl group, an adamantanyl group, a norbornanyl group, a norbornenyl group a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group, each substituted with at least one selected from deuterium, -F,-Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, an adamantanyl group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group; and
-Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), -S(=O)₂(Q₄₀₁), and -P(=O)(Q₄₀₁)(Q₄₀₂), and
Q₄₀₁ to Q₄₀₃ may each independently be selected from a C₁-C₁₀ alkyl group, a C₁-C₁₀ alkoxy group, a phenyl group, a biphenyl group, and a naphthyl group, but are not limited thereto.

In one or more embodiments, when xc1 in Formula 401 is two or more, two A₄₀₁(s) in two or more L₄₀₁(s) may optionally be linked via X₄₀₇, which is a linking group, or two A₄₀₂(s) in two or more L₄₀₁(s) may optionally be linked via X₄₀₈, which is a linking group (see Compounds PD1 to PD4 and PD7). X₄₀₇ and X₄₀₈ may each independently be a single bond, *-O-*', *-S-*', *-C(=O)-*', *-N(Q₄₁₃)-*', *-C(Q₄₁₃)(Q₄₁₄)-*', or *-C(Q₄₁₃)=C(Q₄₁₄)-*' (wherein Q₄₁₃ and Q₄₁₄ may each independently be hydrogen, deuterium, a C₁-C₂₀ alkyl group, a C₁-C₂₀ alkoxy group, a phenyl group, a biphenyl group, a terphenyl group, or a naphthyl group), but embodiments of the present disclosure are not limited thereto.

L₄₀₂ in Formula 401 may be a monovalent, divalent, or trivalent organic ligand. For example, L₄₀₂ may be selected from a halogen, a diketone (for example, acetylacetonate), a carboxylic acid (for example, picolinate), -C(=O), an isonitrile, -CN, and a phosphorus-containing material (for example, phosphine or phosphite), but embodiments of the present disclosure are not limited thereto.

In one or more embodiments, the phosphorescent dopant may be selected from, for example, Compounds PD1 to PD25, but embodiments of the present disclosure are not limited thereto:

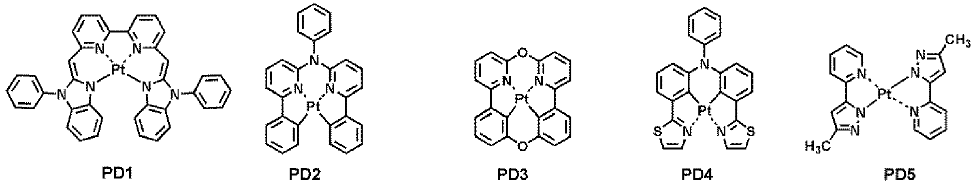

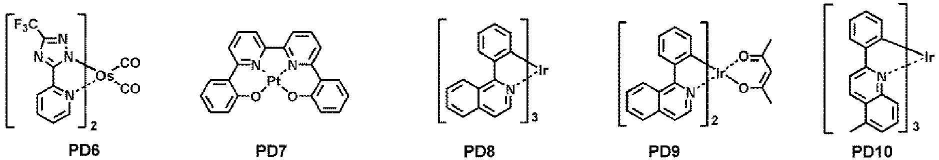

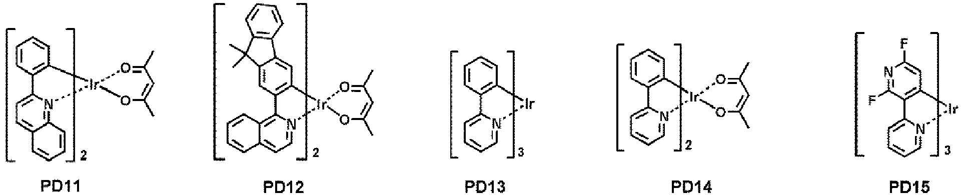

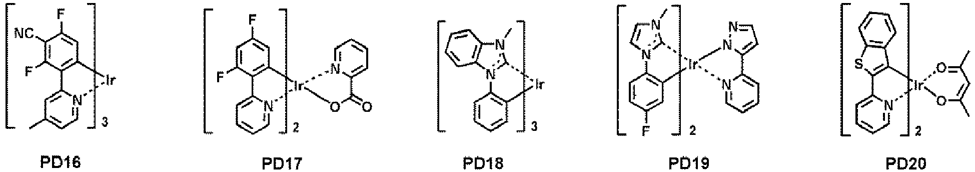

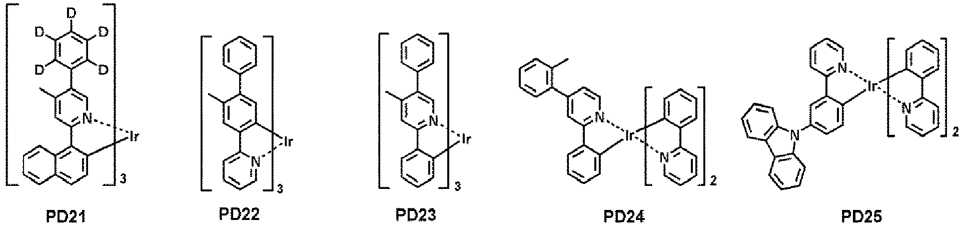

### [Fluorescent dopant in emission layer]

The fluorescent dopant may include an arylamine compound or a styrylamine compound.

The fluorescent dopant may include a compound represented by Formula 501 below:

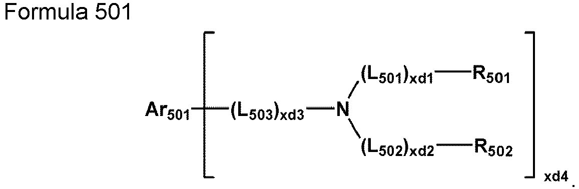

In Formula 501,
Ar₅₀₁ may be a substituted or unsubstituted C₅-C₆₀ carbocyclic group or a substituted or unsubstituted C₁-C₆₀ heterocyclic group,
L₅₀₁ to L₅₀₃ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xd1 to xd3 may each independently be an integer of 0 to 3,
R₅₀₁ and R₅₀₂ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, and
xd4 may be an integer of 1 to 6.

In one embodiment, Ar₅₀₁ in Formula 501 may be selected from:
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group; and
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

In one or more embodiments, L₅₀₁ to L₅₀₃ in Formula 501 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

In one or more embodiments, R₅₀₁ and R₅₀₂ in Formula 501 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, and -Si(Q₃₁)(Q₃₂)(Q₃₃), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In one or more embodiments, xd4 in Formula 501 may be 2, but embodiments of the present disclosure are not limited thereto.

For example, the fluorescent dopant may be selected from Compounds FD1 to FD22:

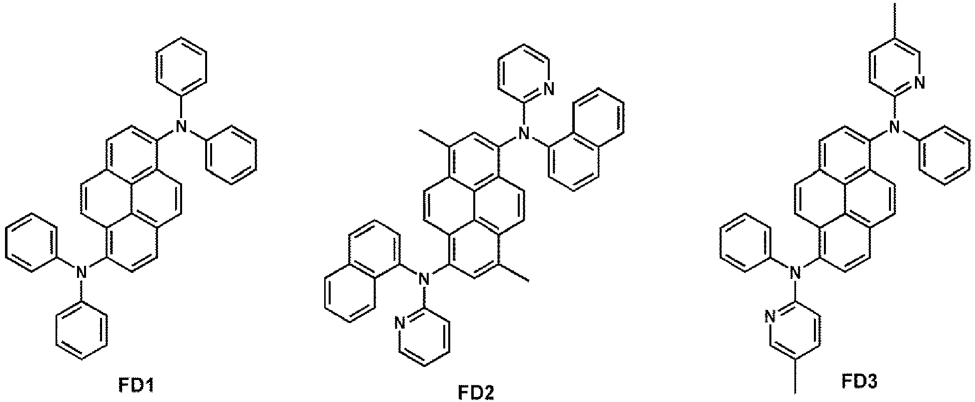

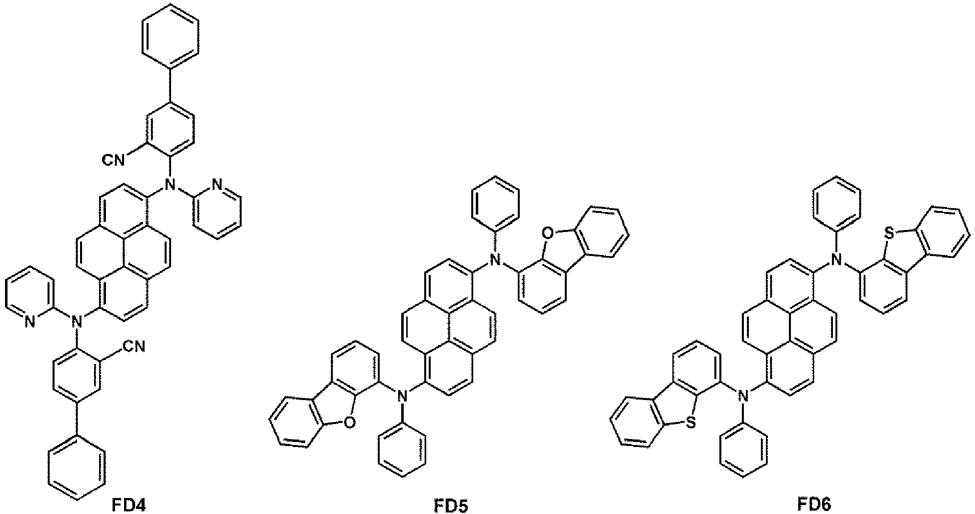

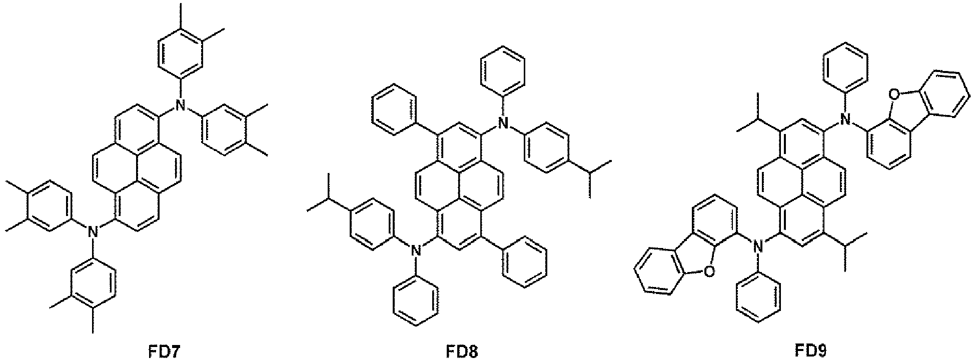

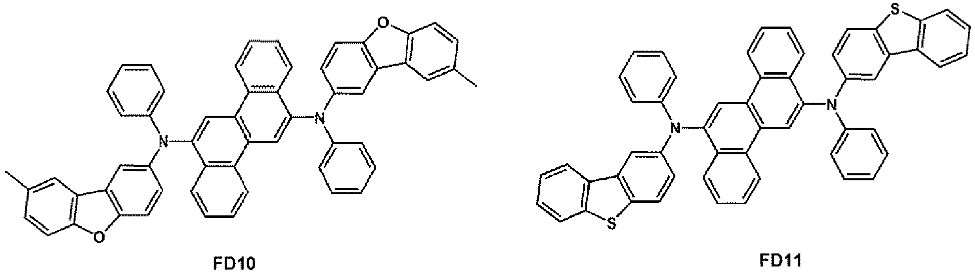

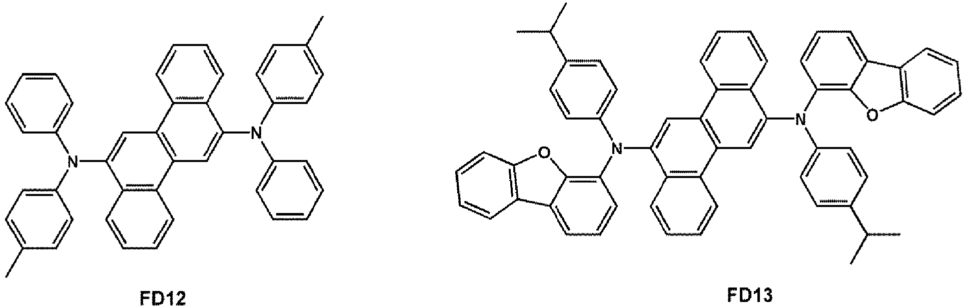

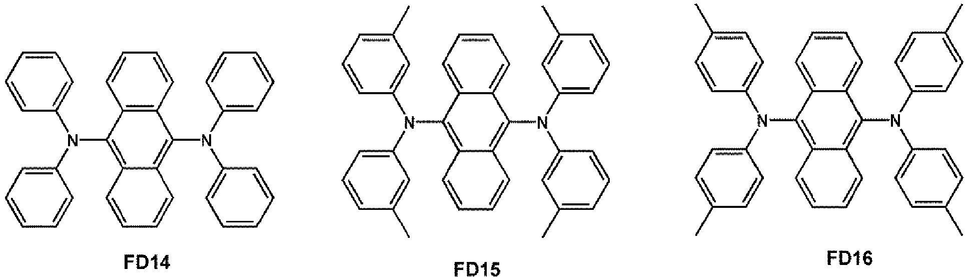

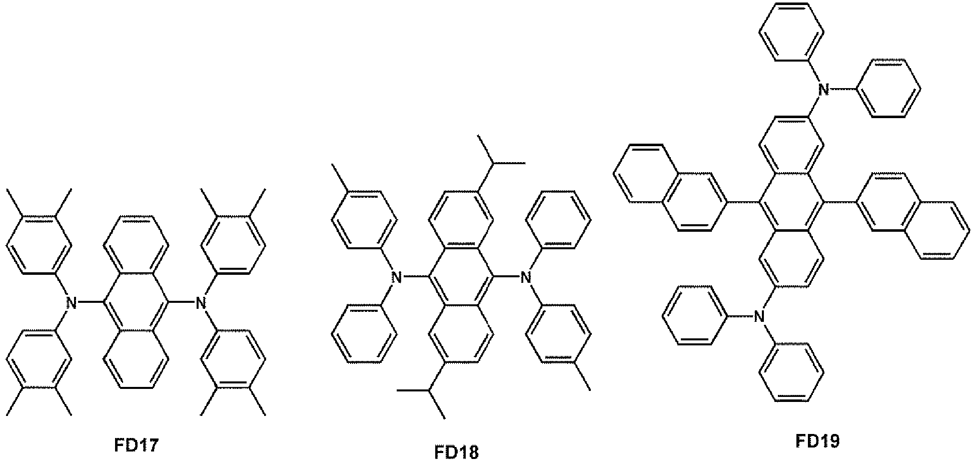

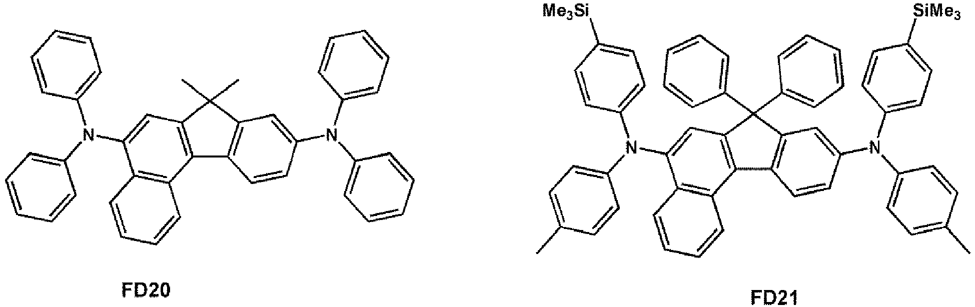

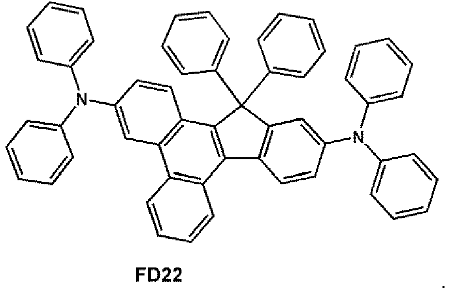

In one or more embodiments, the fluorescent dopant may be selected from the following compounds, but embodiments of the present disclosure are not limited thereto:

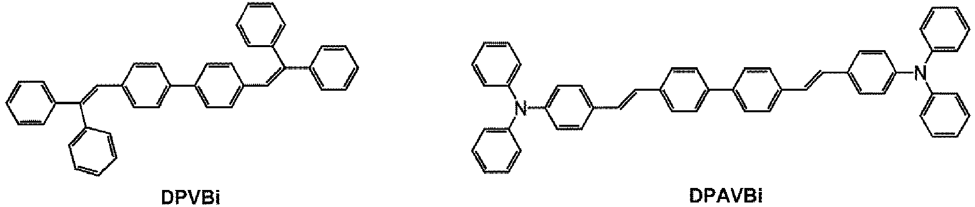

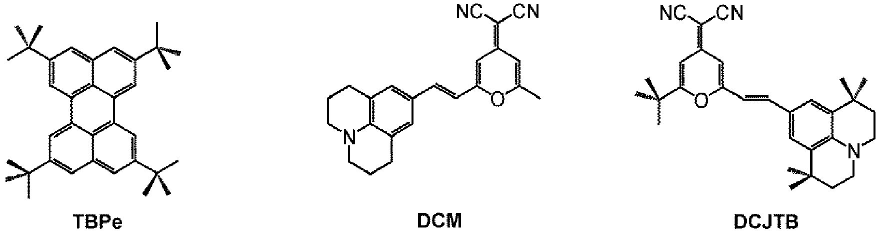

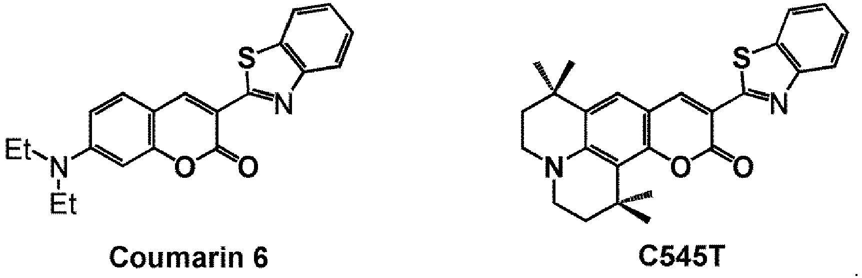

### [Electron transport region in organic layer 150]

The electron transport region may have i) a single-layered structure including a single layer including a single material, ii) a single-layered structure including a single layer including a plurality of different materials, or iii) a multi-layered structure having a plurality of layers including a plurality of different materials.

The electron transport region may include at least one selected from a buffer layer, a hole blocking layer, an electron control layer, an electron transport layer, and an electron injection layer, but embodiments of the present disclosure are not limited thereto.

For example, the electron transport region may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein for each structure, constituting layers are sequentially stacked from an emission layer. However, embodiments of the structure of the electron transport region are not limited thereto.

The electron transport region may include a compound represented by Formula 2, 5, 9, 25, 31, 40, 45, 60, 74, 81, and 90.

In one embodiment, the electron transport region (for example, a buffer layer, a hole blocking layer, an electron control layer, or an electron transport layer in the electron transport region) may include a metal-free compound containing at least one π electron-depleted nitrogen-containing ring.

The term "π electron-depleted nitrogen-containing ring," as used herein, indicates a C₁-C₆₀ heterocyclic group having at least one *-N=*' moiety as a ring-forming moiety.

For example, the "π electron-depleted nitrogen-containing ring" may be i) at least one selected from a 6-membered to 7-membered heteromonocyclic group having at least one *-N=*' moiety, ii) a heteropolycyclic group in which two or more selected from 5-membered to 7-membered heteromonocyclic groups each having at least one *-N=*' moiety are condensed with each other (e.g., combined together), or iii) a heteropolycyclic group in which at least one selected from 5-membered to 7-membered heteromonocyclic groups, each having at least one *-N=*' moiety, is condensed with (e.g., combined with) at least one C₅-C₆₀ carbocyclic group.

Examples of the π electron-depleted nitrogen-containing ring include an imidazole, a pyrazole, a thiazole, an isothiazole, an oxazole, an isoxazole, a pyridine, a pyrazine, a pyrimidine, a pyridazine, an indazole, a purine, a quinoline, an isoquinoline, a benzoquinoline, a phthalazine, a naphthyridine, a quinoxaline, a quinazoline, a cinnoline, a phenanthridine, an acridine, a phenanthroline, a phenazine, a benzimidazole, an isobenzothiazole, a benzoxazole, an isobenzoxazole, a triazole, a tetrazole, an oxadiazole, a triazine, thiadiazol, an imidazopyridine, an imidazopyrimidine, and an azacarbazole, but are not limited thereto.

For example, the electron transport region may include a compound represented by Formula 601:

Formula 601 [Ar₆₀₁]ₓₑ₁₁-[(L₆₀₁)ₓₑ₁-R₆₀₁]ₓₑ₂₁.

In Formula 601,
Ar₆₀₁ may be a substituted or unsubstituted C₅-C₆₀ carbocyclic group or a substituted or unsubstituted C₁-C₆₀ heterocyclic group,
xe11 may be 1, 2, or 3,
L₆₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xe1 may be an integer from 0 to 5,
R₆₀₁ may be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₆₀₁)(Q₆₀₂)(Q₆₀₃),-C(=O)(Q₆₀₁), -S(=O)₂(Q₆₀₁), and -P(=O)(Q₆₀₁)(Q₆₀₂),
Q₆₀₁ to Q₆₀₃ may each independently be a C₁-C₁₀ alkyl group, a C₁-C₁₀ alkoxy group, a phenyl group, a biphenyl group, a terphenyl group, or a naphthyl group, and
xe21 may be an integer from 1 to 5.

In one embodiment, at least one of groups Ar₆₀₁ in the number of xe11 and groups R₆₀₁ in the number of xe21 may include the π electron-depleted nitrogen-containing ring.

In one embodiment, ring Ar₆₀₁ in Formula 601 may be selected from:
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an iso-benzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group; and
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an iso-benzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -S(=O)₂(Q₃₁), and -P(=O)(Q₃₁)(Q₃₂), and
Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

When xe11 in Formula 601 is two or more, two or more Ar₆₀₁(s) may be linked via a single bond.

In one or more embodiments, Ar₆₀₁ in Formula 601 may be an anthracene group.

In one or more embodiments, a compound represented by Formula 601 may be represented by Formula 601-1:

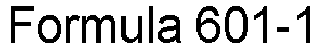

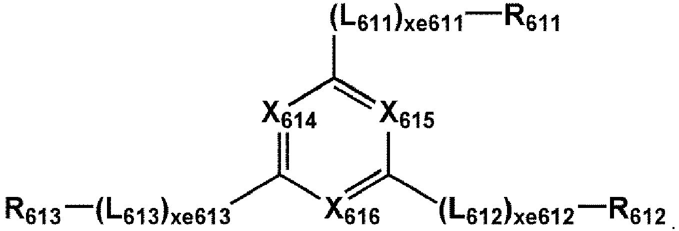

In Formula 601-1,
X₆₁₄ may be N or C(R₆₁₄), X₆₁₅ may be N or C(R₆₁₅), X₆₁₆ may be N or C(R₆₁₆), and at least one selected from X₆₁₄ to X₆₁₆ may be N,
L₆₁₁ to L₆₁₃ may each independently be the same as described in connection with L₆₀₁,
xe611 to xe613 may each independently be the same as described in connection with xe1,
R₆₁₁ to R₆₁₃ may each independently be the same as described in connection with R₆₀₁, and
R₆₁₄ to R₆₁₆ may each independently be selected from hydrogen, deuterium, -F,-Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

In one embodiment, L₆₀₁ and L₆₁₁ to L₆₁₃ in Formulae 601 and 601-1 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, an isobenzoxazolylene group, a triazolylene group, a tetrazolylene group, an imidazopyridinylene group, an imidazopyrimidinylene group, and an azacarbazolylene group, each substituted with at least one selected from deuterium, -F,-CI, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group,
but embodiments of the present disclosure are not limited thereto.

In one or more embodiments, xe1 and xe611 to xe613 in Formulae 601 and 601-1 may each independently be 0, 1, or 2.

In one or more embodiments, in Formulae 601 and 601-1, R₆₀₁ and R₆₁₁ to R₆₁₃ may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group;
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
-S(=O)₂(Q₆₀₁) and -P(=O)(Q₆₀₁)(Q₆₀₂), and
Q₆₀₁ and Q₆₀₂ may be the same as described above.

The electron transport region may include at least one compound selected from Compounds ET1 to ET36, but embodiments of the present disclosure are not limited thereto:

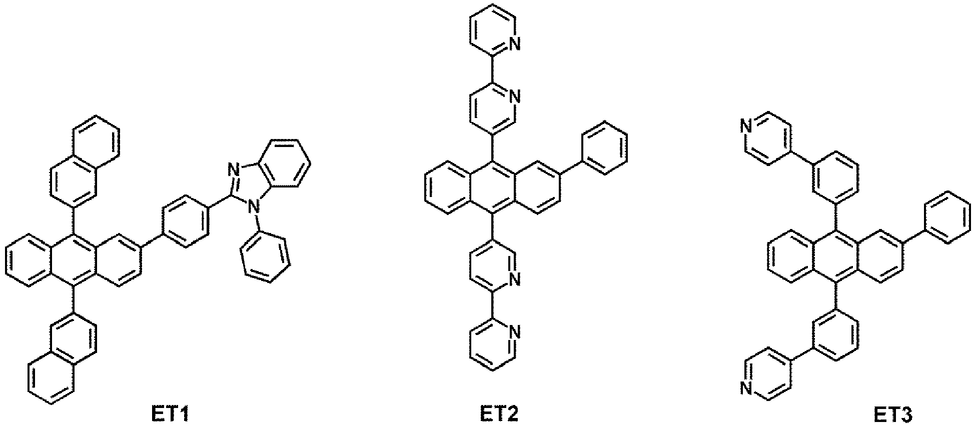

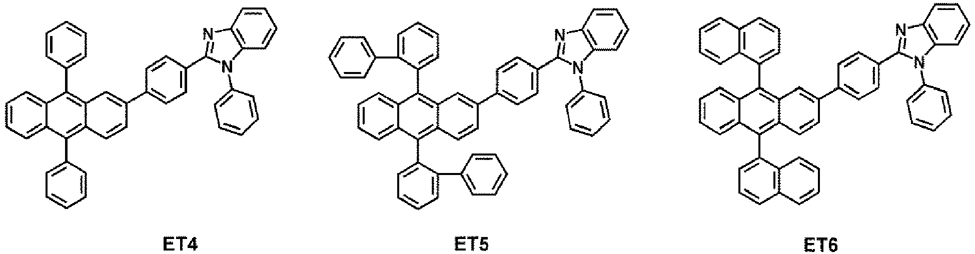

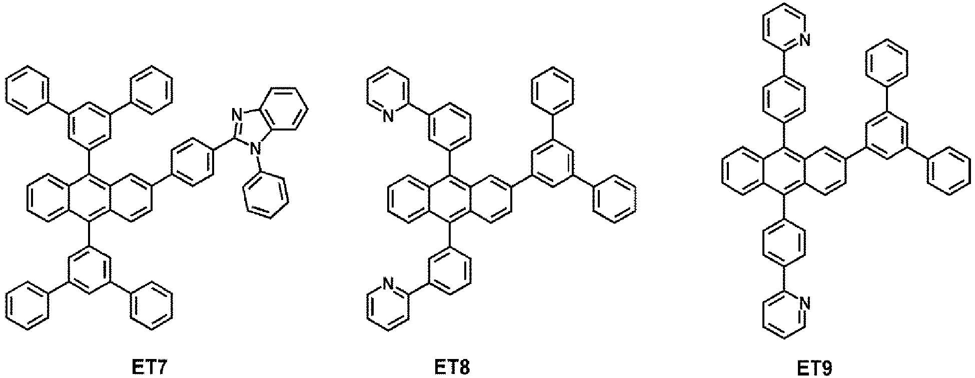

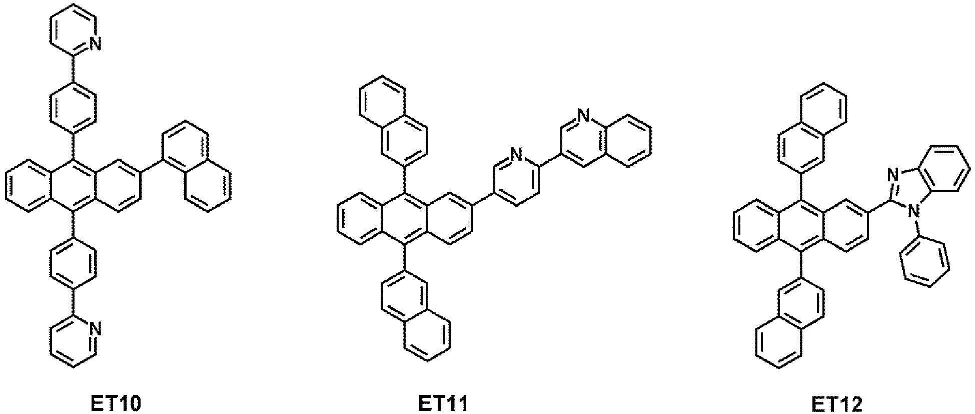

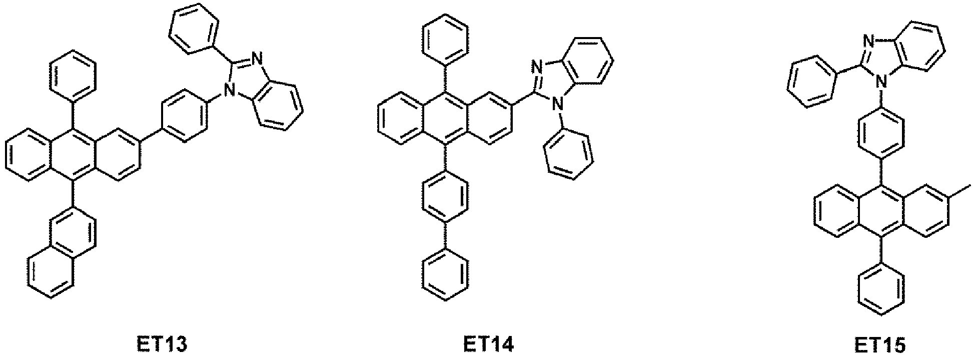

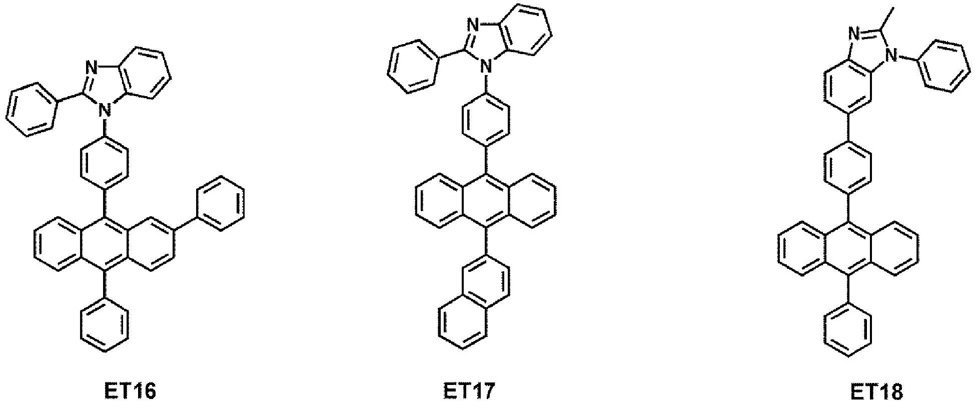

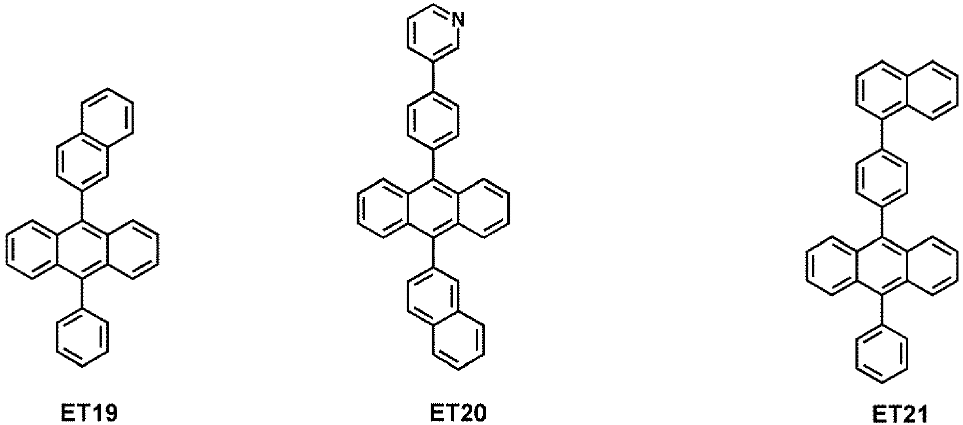

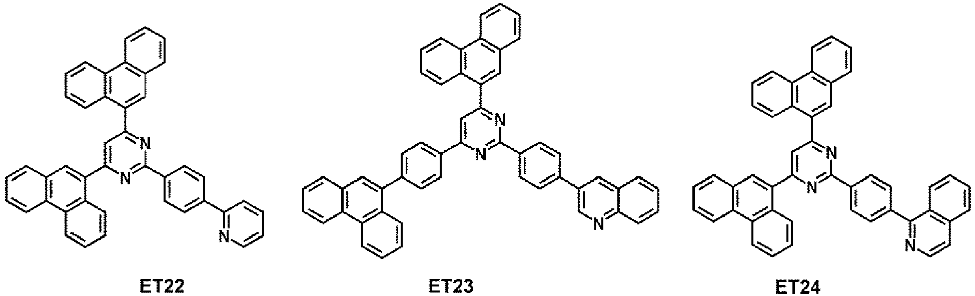

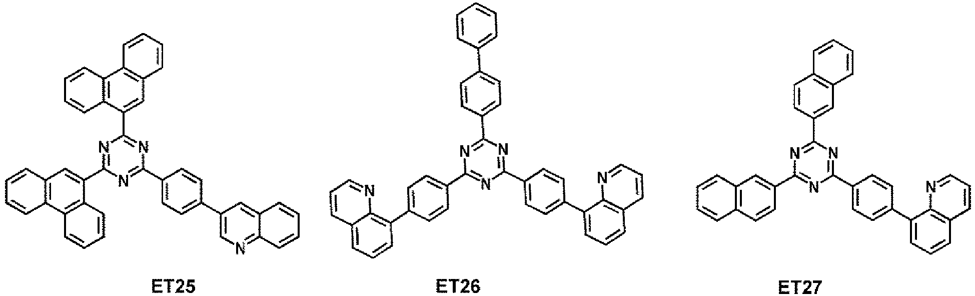

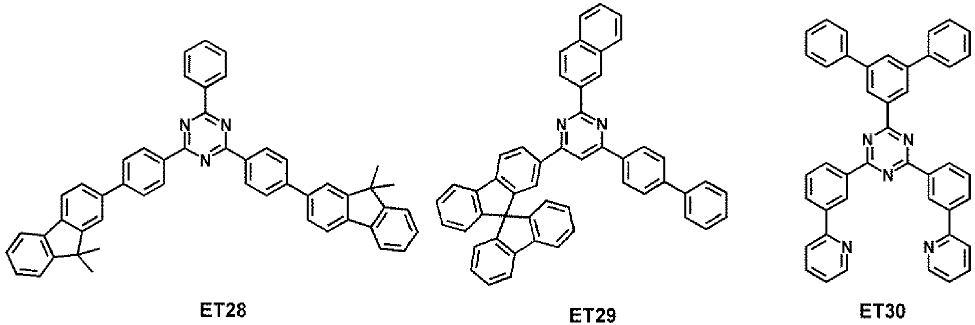

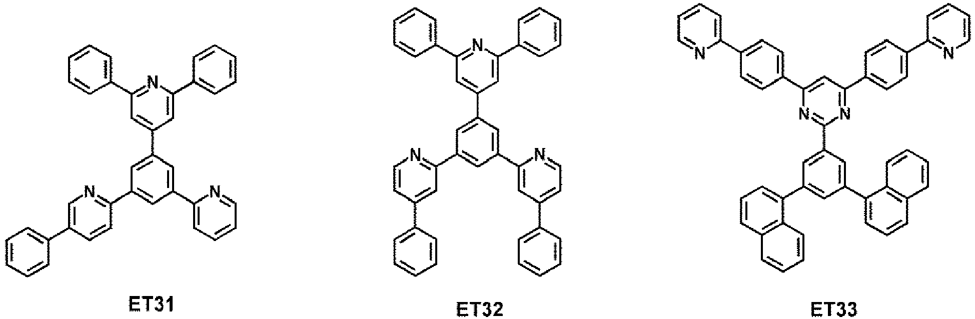

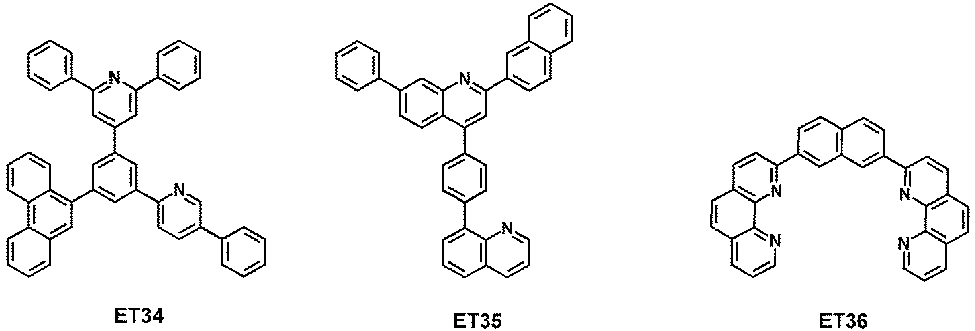

In one or more embodiments, the electron transport region may include at least one selected from 2,9-dimethyl-4,7-diphenyl-1,10-phenanthroline (BCP), 4,7-diphenyl-1,10-phenanthroline (Bphen), Alq₃, BAlq, 3-(biphenyl-4-yl)-5-(4-tert-butylphenyl)-4-phenyl-4H-1,2,4-triazole (TAZ), and NTAZ:

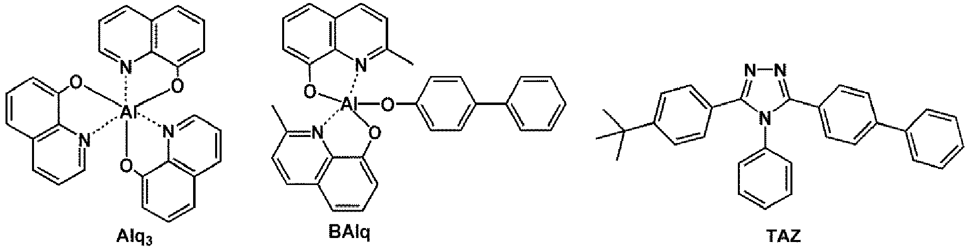

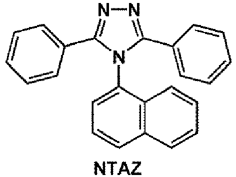

Thicknesses of the buffer layer, the hole blocking layer, and the electron control layer may each be in a range of about 20 Å to about 1,000 Å, for example, about 30 Å to about 300 Å. When the thicknesses of the buffer layer, the hole blocking layer, and the electron control layer are within these ranges, the electron blocking layer may have excellent electron blocking characteristics or electron control characteristics without a substantial increase in driving voltage.

A thickness of the electron transport layer may be in a range of about 100 Å to about 1,000 Å, for example, about 150 Å to about 500 Å. When the thickness of the electron transport layer is within the range described above, the electron transport layer may have suitable or satisfactory electron transport characteristics without a substantial increase in driving voltage.

The electron transport region (for example, the electron transport layer in the electron transport region) may further include, in addition to the materials described above, a metal-containing material.

The metal-containing material may include at least one selected from alkali metal complex and alkaline earth-metal complex. The alkali metal complex may include a metal ion selected from a Li ion, a Na ion, a K ion, a Rb ion, and a Cs ion, and the alkaline earth-metal complex may include a metal ion selected from a Be ion, a Mg ion, a Ca ion, a Sr ion, and a Ba ion. A ligand coordinated with the metal ion of the alkali metal complex or the alkaline earth-metal complex may be selected from a hydroxy quinoline, a hydroxy isoquinoline, a hydroxy benzoquinoline, a hydroxy acridine, a hydroxy phenanthridine, a hydroxy phenyloxazole, a hydroxy phenylthiazole, a hydroxy diphenyloxadiazole, a hydroxy diphenylthiadiazol, a hydroxy phenylpyridine, a hydroxy phenylbenzimidazole, a hydroxy phenylbenzothiazole, a bipyridine, a phenanthroline, and a cyclopentadiene, but embodiments of the present disclosure are not limited thereto.

For example, the metal-containing material may include a Li complex. The Li complex may include, for example, Compound ET-D1 (lithium quinolate, LiQ) or ET-D2:

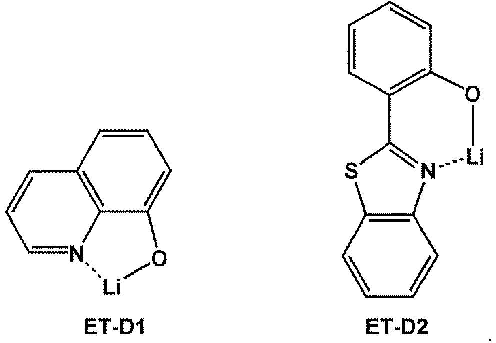

The electron transport region may include an electron injection layer that facilitates injection of electrons from the second electrode 190. The electron injection layer may directly contact the second electrode 190.

The electron injection layer may have i) a single-layered structure including a single layer including a single material, ii) a single-layered structure including a single layer including a plurality of different materials, or iii) a multi-layered structure having a plurality of layers including a plurality of different materials.

The electron injection layer may include an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof.

The alkali metal may be selected from Li, Na, K, Rb, and Cs. In one embodiment, the alkali metal may be Li, Na, or Cs. In one or more embodiments, the alkali metal may be Li or Cs, but embodiments of the present disclosure are not limited thereto.

The alkaline earth metal may be selected from Mg, Ca, Sr, and Ba.

The rare earth metal may be selected from Sc, Y, Ce, Tb, Yb, and Gd.

The alkali metal compound, the alkaline earth-metal compound, and the rare earth metal compound may be selected from oxides and halides (for example, fluorides, chlorides, bromides, or iodides) of the alkali metal, the alkaline earth-metal, and the rare earth metal.

The alkali metal compound may be selected from alkali metal oxides, such as Li₂O, Cs₂O, or K₂O, and alkali metal halides, such as LiF, NaF, CsF, KF, Lil, Nal, Csl, or KI. In one embodiment, the alkali metal compound may be selected from LiF, Li₂O, NaF, Lil, Nal, Csl, and KI, but embodiments of the present disclosure are not limited thereto.

The alkaline earth metal compound may be selected from BaO, SrO, CaO, BaₓSr₁₋ₓO (0<x<1), and BaₓCa₁₋ₓO (0<x<1). In one embodiment, the alkaline earth metal compound may be selected from BaO, SrO, and CaO, but embodiments of the present disclosure are not limited thereto.

The rare earth metal compound may be selected from YbF₃, ScF₃, ScO₃, Y₂O₃, Ce₂O₃, GdF₃, and TbF₃. In one embodiment, the rare earth metal compound may be selected from YbF₃, ScF₃, TbF₃, YbI₃, ScI₃, and TbI₃, but embodiments of the present disclosure are not limited thereto.

The alkali metal complex, the alkaline earth-metal complex, and the rare earth metal complex may include an ion of alkali metal, alkaline earth-metal, and rare earth metal as described above, and a ligand coordinated with a metal ion of the alkali metal complex, the alkaline earth-metal complex, or the rare earth metal complex may be selected from hydroxy quinoline, hydroxy isoquinoline, hydroxy benzoquinoline, hydroxy acridine, hydroxy phenanthridine, hydroxy phenyl oxazole, hydroxy phenylthiazole, hydroxy diphenyl oxadiazole, hydroxy diphenylthiadiazol, hydroxy phenylpyridine, hydroxy phenylbenzimidazole, hydroxy phenylbenzothiazole, bipyridine, phenanthroline, and cyclopentadiene, but embodiments of the present disclosure are not limited thereto.

The electron injection layer may consist of an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof, as described above. In one or more embodiments, the electron injection layer may further include an organic material. When the electron injection layer further includes an organic material, an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof may be homogeneously or non-homogeneously dispersed in a matrix including the organic material.

A thickness of the electron injection layer may be in a range of about 1 ÅÅ to about 100 ÅÅ, for example, about 3 ÅÅ to about 90 ÅÅ. When the thickness of the electron injection layer is within the range described above, the electron injection layer may have suitable or satisfactory electron injection characteristics without a substantial increase in driving voltage.

### [Second electrode 190]

The second electrode 190 may be disposed on the organic layer 150 having such a structure. The second electrode 190 may be a cathode which is an electron injection electrode, and in this regard, a material for forming the second electrode 190 may be selected from metal, an alloy, an electrically conductive compound, and a combination thereof, which have a relatively low work function.

The second electrode 190 may include at least one selected from lithium (Li), silver (Ag), magnesium (Mg), aluminum (Al), aluminum-lithium (Al-Li), calcium (Ca), magnesium-indium (Mg-In), magnesium-silver (Mg-Ag), ITO, and IZO, but embodiments of the present disclosure are not limited thereto. The second electrode 190 may be a transmissive electrode, a semi-transmissive electrode, or a reflective electrode.

The second electrode 190 may have a single-layered structure, or a multilayered structure including two or more layers.

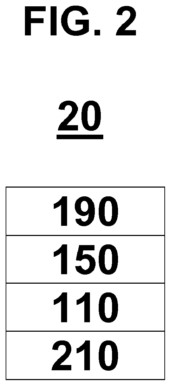

### Description of FIGS. 2 to 4

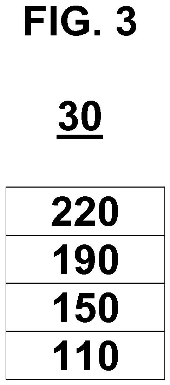

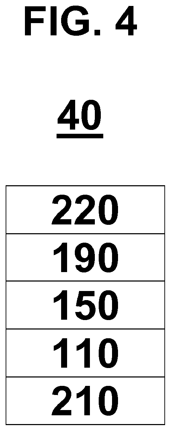

An organic light-emitting device 20 of FIG. 2 includes a first capping layer 210, a first electrode 110, an organic layer 150, and a second electrode 190 which are sequentially stacked in this stated order, an organic light-emitting device 30 of FIG. 3 includes a first electrode 110, an organic layer 150, a second electrode 190, and a second capping layer 220 which are sequentially stacked in this stated order, and an organic light-emitting device 40 of FIG. 4 includes a first capping layer 210, a first electrode 110, an organic layer 150, a second electrode 190, and a second capping layer 220, wherein at least one of the organic layer and the capsulating layers include a compound represented by formula 2, 5, 9, 25, 31, 40, 45, 60, 74, 81, or 90.

Regarding FIGS. 2 to 4, the first electrode 110, the organic layer 150, and the second electrode 190 may be understood by referring to the description presented in connection with FIG. 1.

In the organic layer 150 of each of the organic light-emitting devices 20 and 40, light generated in an emission layer may pass through the first electrode 110, which is a semi-transmissive electrode or a transmissive electrode, and the first capping layer 210 toward the outside, and in the organic layer 150 of each of the organic light-emitting devices 30 and 40, light generated in an emission layer may pass through the second electrode 190, which is a semi-transmissive electrode or a transmissive electrode, and the second capping layer 220 toward the outside.

The first capping layer 210 and the second capping layer 220 may increase external luminescent efficiency according to the principle of constructive interference.

The first capping layer 210 and the second capping layer 220 may each independently be an organic capping layer including an organic material, an inorganic capping layer including an inorganic material, or a composite capping layer including an organic material and an inorganic material.

At least one selected from the first capping layer 210 and the second capping layer 220 may each independently include at least one material selected from carbocyclic compounds, heterocyclic compounds, amine-based compounds, porphyrine derivatives, phthalocyanine derivatives, naphthalocyanine derivatives, alkali metal complexes, and alkaline earth-based complexes. The carbocyclic compound, the heterocyclic compound, and the amine-based compound may be optionally substituted with a substituent containing at least one element selected from O, N, S, Se, Si, F, Cl, Br, and I.

In one embodiment, at least one selected from the first capping layer 210 and the second capping layer 220 may each independently include an amine-based compound.

In one embodiment, at least one selected from the first capping layer 210 and the second capping layer 220 may each independently include the compound represented by Formula 201 or the compound represented by Formula 202.

In one or more embodiments, at least one selected from the first capping layer 210 and the second capping layer 220 may each independently include a compound selected from Compounds HT28 to HT33 (shown elsewhere herein) and Compounds CP1 to CP5, but embodiments of the present disclosure are not limited thereto:

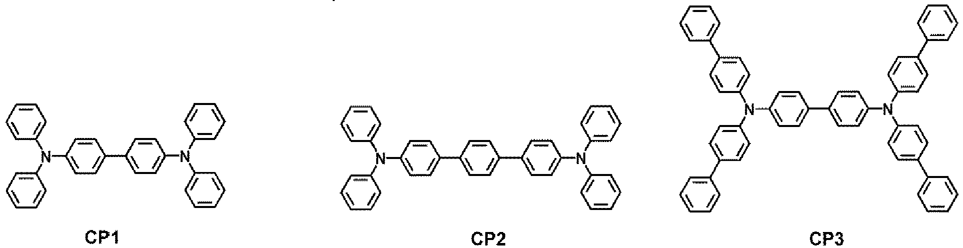

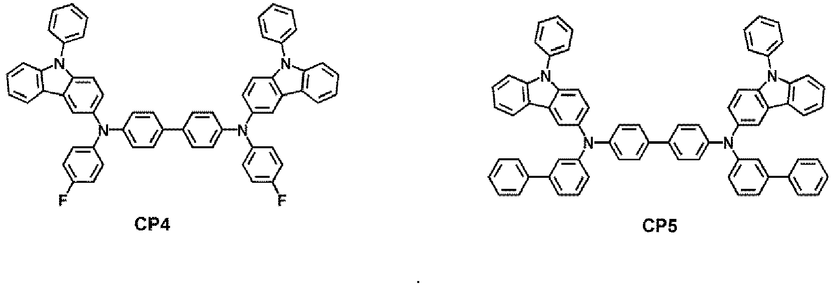

Hereinbefore, the organic light-emitting device according to an embodiment has been described in connection with FIGS. 1 to 4. However, embodiments of the present disclosure are not limited thereto.

Layers constituting the hole transport region, an emission layer, and layers constituting the electron transport region may be formed in a certain region by using one or more suitable methods selected from vacuum deposition, spin coating, casting, Langmuir-Blodgett (LB) deposition, ink-jet printing, laser-printing, and laser-induced thermal imaging.

When layers constituting the hole transport region, an emission layer, and layers constituting the electron transport region are formed by vacuum deposition, the vacuum deposition may be performed at a deposition temperature of about 100 °C to about 500 °C , at a vacuum degree of about 10⁻⁸ to torr to about 10⁻³ torr, and at a deposition speed of about 0.01 Å/sec to about 100 Å/sec by taking into account a material to be included in a layer to be formed, and the structure of a layer to be formed.

When layers constituting the hole transport region, an emission layer, and layers constituting the electron transport region are formed by spin coating, the spin coating may be performed at a coating speed of about 2,000 rpm to about 5,000 rpm and at a heat treatment temperature of about 80 °C to 200 °C by taking into account a material to be included in a layer to be formed, and the structure of a layer to be formed.

### [General definition of substituents]

The term "C₁-C₆₀ alkyl group," as used herein, refers to a linear or branched aliphatic saturated hydrocarbon monovalent group having 1 to 60 carbon atoms, and examples thereof include a methyl group, an ethyl group, a propyl group, an isobutyl group, a sec-butyl group, a tert-butyl group, a pentyl group, an iso-amyl group, and a hexyl group. The term "C₁-C₆₀ alkylene group," as used herein, refers to a divalent group having substantially the same structure as the C₁-C₆₀ alkyl group, except that the C₁-C₆₀ alkylene group is divalent instead of monovalent.

The term "C₂-C₆₀ alkenyl group," as used herein, refers to a hydrocarbon group having at least one carbon-carbon double bond at a main chain (e.g., in the middle) or at a terminus of the C₂-C₆₀ alkyl group, and examples thereof include an ethenyl group, a propenyl group, and a butenyl group. The term "C₂-C₆₀ alkenylene group," as used herein, refers to a divalent group having substantially the same structure as the C₂-C₆₀ alkenyl group, except that the C₂-C₆₀ alkenylene group is divalent instead of monovalent.

The term "C₂-C₆₀ alkynyl group," as used herein, refers to a hydrocarbon group having at least one carbon-carbon triple bond at a main chain (e.g., in the middle) or at a terminus of the C₂-C₆₀ alkyl group, and examples thereof include an ethynyl group, and a propynyl group. The term "C₂-C₆₀ alkynylene group," as used herein, refers to a divalent group having substantially the same structure as the C₂-C₆₀ alkynyl group, except that the C₂-C₆₀ alkynylene group is divalent instead of monovalent.

The term "C₁-C₆₀ alkoxy group," as used herein, refers to a monovalent group represented by -OA₁₀₁ (wherein A₁₀₁ is the C₁-C₆₀ alkyl group), and examples thereof include a methoxy group, an ethoxy group, and an isopropyloxy group.

The term "C₃-C₁₀ cycloalkyl group," as used herein, refers to a monovalent saturated hydrocarbon monocyclic group having 3 to 10 carbon atoms, and examples thereof include a cyclopropyl group, a cyclobutyl group, a cyclopentyl group, a cyclohexyl group, and a cycloheptyl group. The term "C₃-C₁₀ cycloalkylene group," as used herein, refers to a divalent group having substantially the same structure as the C₃-C₁₀ cycloalkyl group, except that the C₃-C₁₀ cycloalkylene group is divalent instead of monovalent.

The term "C₁-C₁₀ heterocycloalkyl group," as used herein, refers to a monovalent monocyclic group having at least one heteroatom selected from N, O, Si, P, and S as a ring-forming atom and 1 to 10 carbon atoms, and examples thereof include a 1,2,3,4-oxatriazolidinyl group, a tetrahydrofuranyl group, and a tetrahydrothiophenyl group. The term "C₁-C₁₀ heterocycloalkylene group," as used herein, refers to a divalent group having substantially the same structure as the C₁-C₁₀ heterocycloalkyl group, except that the C₁-C₁₀ heterocycloalkylene group is divalent instead of monovalent.

The term "C₃-C₁₀ cycloalkenyl group," as used herein, refers to a monovalent monocyclic group that has 3 to 10 carbon atoms and at least one carbon-carbon double bond in the ring thereof and no aromaticity (e.g., the monocyclic group is not aromatic), and examples thereof include a cyclopentenyl group, a cyclohexenyl group, and a cycloheptenyl group. The term "C₃-C₁₀ cycloalkenylene group," as used herein, refers to a divalent group having substantially the same structure as the C₃-C₁₀ cycloalkenyl group, except that the C₃-C₁₀ cycloalkenylene group is divalent instead of monovalent.

The term "C₁-C₁₀ heterocycloalkenyl group," as used herein, refers to a monovalent monocyclic group that has at least one heteroatom selected from N, O, Si, P, and S as a ring-forming atom, 1 to 10 carbon atoms, and at least one carbon-carbon double bond in its ring. Non-limiting examples of the C₁-C₁₀ heterocycloalkenyl group include a 4,5-dihydro-1,2,3,4-oxatriazolyl group, a 2,3-dihydrofuranyl group, and a 2,3-dihydrothiophenyl group. The term "C₁-C₁₀ heterocycloalkenylene group," as used herein, refers to a divalent group having substantially the same structure as the C₁-C₁₀ heterocycloalkenyl group, except that the C₁-C₁₀ heterocycloalkenylene group is divalent instead of monovalent.

The term "C₆-C₆₀ aryl group," as used herein, refers to a monovalent group having a carbocyclic aromatic system having 6 to 60 carbon atoms, and a C₆-C₆₀ arylene group used herein refers to a divalent group having a carbocyclic aromatic system having 6 to 60 carbon atoms. Non-limiting examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other (e.g., combined together).

The term "C₁-C₆₀ heteroaryl group," as used herein, refers to a monovalent group having a carbocyclic aromatic system that has at least one heteroatom selected from N, O, Si, P, and S as a ring-forming atom, in addition to 1 to 60 carbon atoms. The term "C₁-C₆₀ heteroarylene group," as used herein, refers to a divalent group having a carbocyclic aromatic system that has at least one heteroatom selected from N, O, Si, P, and S as a ring-forming atom, in addition to 1 to 60 carbon atoms. Non-limiting examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be condensed with each other (e.g., combined together).

The term "C₆-C₆₀ aryloxy group," as used herein, refers to -OA₁₀₂ (wherein A₁₀₂ is the C₆-C₆₀ aryl group), and a C₆-C₆₀ arylthio group used herein indicates -SA₁₀₃ (wherein A₁₀₃ is the C₆-C₆₀ aryl group).

The term "monovalent non-aromatic condensed polycyclic group," as used herein, refers to a monovalent group (for example, having 8 to 60 carbon atoms) having two or more rings condensed with each other (e.g., combined together), only carbon atoms as ring-forming atoms, and no aromaticity in its entire molecular structure (e.g., the entire molecule is not aromatic). A detailed example of the monovalent non-aromatic condensed polycyclic group is a fluorenyl group. The term "divalent non-aromatic condensed polycyclic group," used herein, refers to a divalent group having substantially the same structure as the monovalent non-aromatic condensed polycyclic group, except that the divalent non-aromatic condensed polycyclic group is divalent instead of monovalent.

The term "monovalent non-aromatic condensed heteropolycyclic group," as used herein, refers to a monovalent group (for example, having 1 to 60 carbon atoms) having two or more rings condensed to each other (e.g., combined together), at least one heteroatom selected from N, O, Si, P, and S, other than carbon atoms, as a ring-forming atom, and no aromaticity in its entire molecular structure (e.g., the entire molecule is not aromatic). An example of the monovalent non-aromatic condensed heteropolycyclic group is a carbazolyl group. The term "divalent non-aromatic condensed heteropolycyclic group," as used herein, refers to a divalent group having substantially the same structure as the monovalent non-aromatic condensed heteropolycyclic group, except that the divalent non-aromatic condensed heteropolycyclic group is divalent instead of monovalent.

The term "C₅-C₆₀ carbocyclic group," as used herein, refers to a monocyclic or polycyclic group having 5 to 60 carbon atoms in which a ring-forming atom is a carbon atom only (e.g., the ring itself only includes carbon atoms). The C₅-C₆₀ carbocyclic group may be an aromatic carbocyclic group or a non-aromatic carbocyclic group. The C₅-C₆₀ carbocyclic group may be a ring, such as benzene, a monovalent group, such as a phenyl group, or a divalent group, such as a phenylene group. In one or more embodiments, depending on the number of substituents connected to the C₅-C₆₀ carbocyclic group, the C₅-C₆₀ carbocyclic group may be a trivalent group or a quadrivalent group.

The term "C₁-C₆₀ heterocyclic group," as used herein, refers to a group having the same structure as the C₁-C₆₀ carbocyclic group, except that as a ring-forming atom, at least one heteroatom selected from N, O, Si, P, and S is used in addition to carbon (the number of carbon atoms may be in a range of 1 to 60).

At least one substituent of the substituted C₅-C₆₀ carbocyclic group, the substituted C₁-C₆₀ heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may be selected from:

deuterium (-D), -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl, -Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁) and-P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₂₁)(Q₂₂)(Q₂₃),-N(Q₂₁)(Q₂₂), -B(Q₂₁)(Q₂₂), -C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and-P(=O)(Q₃₁)(Q₃₂), and
Q₁₁ to Q₁₃, Q₂₁ to Q₂₃, and Q₃₁ to Q₃₃ may each independently be selected from hydrogen, deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, a biphenyl group, and a terphenyl group.

The term "Ph," as used herein, refers to a phenyl group, the term "Me," as used herein, refers to a methyl group, the term "Et," as used herein, refers to an ethyl group, the term "ter-Bu" or "Bu^{t}," as used herein, refers to a tert-butyl group, and the term "OMe," as used herein, refers to a methoxy group.

The term "biphenyl group," as used herein, refers to "a phenyl group substituted with a phenyl group." In other words, the "biphenyl group" is a substituted phenyl group having a C₆-C₆₀ aryl group as a substituent.

The term "terphenyl group," as used herein, refers to "a phenyl group substituted with a biphenyl group." In other words, the "terphenyl group" is a phenyl group having, as a substituent, a C₆-C₆₀ aryl group substituted with a C₆-C₆₀ aryl group.
* and *' used herein, unless defined otherwise, each refer to a binding site to a neighboring atom in a corresponding formula.

Hereinafter, a compound according to embodiments and an organic light-emitting device according to embodiments will be described in more detail with reference to Synthesis Examples and Examples. The wording "B was used instead of A" used in describing Synthesis Examples refers to that an identical (e.g., substantially identical) molar equivalent of B was used in place of A.

### Examples

### Synthesis Example 1: Synthesis of Compound 2

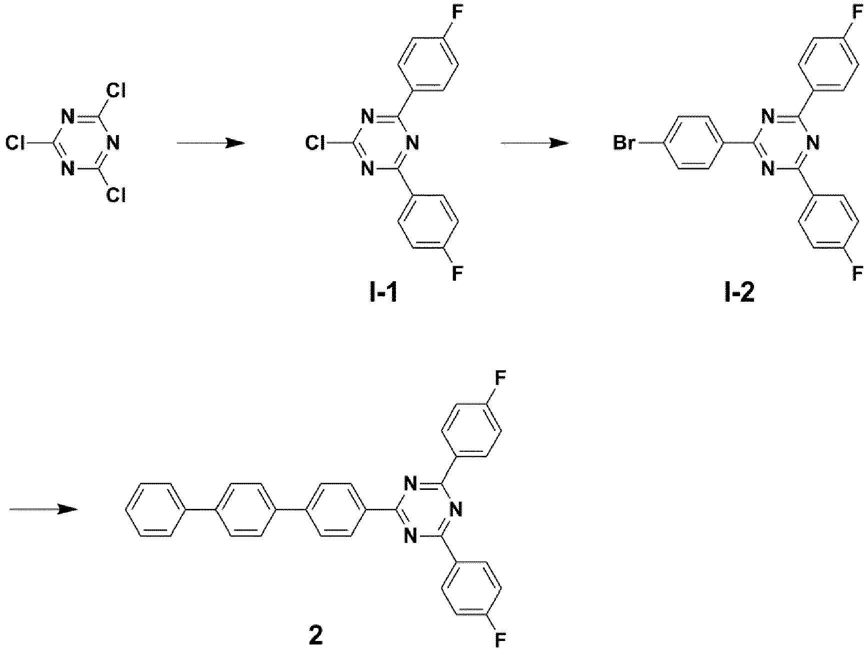

### Synthesis of Intermediate I-1

1.84 g (10 mmol) of cyanuric chloride was dissolved in 30 mL of tetrahydrofuran (THF), 26 mL of 4-fluorophenyl magnesium bromide (1.0 M in THF) was added thereto at a temperature of 0 °C, and the reaction solution was stirred at a temperature of 35 °C for 12 hours. Then, the reaction solution was cooled to room temperature, and an organic layer was extracted therefrom three times by using 60 mL of water and 60 mL of diethylether. The extracted organic layer was dried by using magnesium sulfate, and a solvent was evaporated therefrom. Then, the residue obtained therefrom was separated and purified by silica gel column chromatography to obtain 1.36 g (yield: 45%) of Intermediate I-1. The obtained compound was identified by LC-MS.

C₁₅H₈ClF₂N₃: M+1 303.4.

### Synthesis of Intermediate I-2

3.34 g (11.0 mmol) of Intermediate I-1, 2.00 g (10.0 mmol) of 4-bromophenyl boronic acid, 0.58 g (0.50 mmol) of Pd(PPh₃)₄, and 4.15 g (30.0 mmol) of K₂CO₃ were dissolved in 60 mL of a mixed solution including THF and H₂O (2/1) and stirred at a temperature of 80 °C for 16 hours. Then, the reaction solution was cooled to room temperature, and an organic layer was extracted therefrom three times by using 60 mL of water and 60 mL of diethylether. The extracted organic layer was dried by using magnesium sulfate, and a solvent was evaporated therefrom. Then, the residue obtained therefrom was separated and purified by silica gel column chromatography to obtain 2.12 g (yield: 50%) of Intermediate I-2. The obtained compound was identified by LC-MS.

C₂₁H₁₂BrF₂N₃: M+1 423.0.

### Synthesis of Compound 2

4.23 g (10 mmol) of Intermediate I-2, 1.98 g (10 mmol) of [1,1'-biphenyl]-4-ylboronic acid, 0.58 g (0.5 mmol) of Pd(PPh₃)₄, and 4.14 g (30 mmol) of K₂CO₃ were dissolved in 60 mL of a mixed solution including THF and H₂O (2/1) and stirred at a temperature of 80 °C for 16 hours. Then, the reaction solution was cooled to room temperature, and an organic layer was extracted therefrom three times by using 40 mL of water and 50 mL of ethylether. The extracted organic layer was dried by using magnesium sulfate, and a solvent was evaporated therefrom. Then, the residue obtained therefrom was separated and purified by silica gel column chromatography to obtain 4.08 g (yield: 82%) of Compound 2. The obtained compound was identified by MS/FAB and ¹H NMR.

C₃₃H₂₁F₂N₃ cal. 497.17, found 497.16.

### Synthesis Example 2: Synthesis of Compound 5

5.17 g (yield: 83%) of Compound 5 was synthesized in substantially the same manner as in Synthesis of Compound 2, except that (4-(4-phenylnaphthalen-1-yl)phenyl)boronic acid was used instead of [1,1'-biphenyl]-4-ylboronic acid. The obtained compound was identified by MS/FAB and ¹H NMR.

C₄₃H₂₇F₂N₃ cal. 623.22, found 623.23.

### Synthesis Example 3: Synthesis of Compound 9

4.73 g (yield: 76%) of Compound 9 was synthesized in substantially the same manner as in Synthesis of Compound 2, except that (6-([1,1'-biphenyl]-4-yl)naphthalen-2-yl)boronic acid was used instead of [1,1'-biphenyl]-4-ylboronic acid. The obtained compound was identified by MS/FAB and ¹H NMR.

C₄₃H₂₇F₂N₃ cal. 623.22, found 623.21.

### Synthesis Example 4: Synthesis of Compound 25

4.42 g (yield: 72%) of Compound 25 was synthesized in substantially the same manner as in Synthesis of Compound 2, except that (9,9-dimethyl-7-phenyl-9H-fluoren-2-yl)boronic acid was used instead of [1,1'-biphenyl]-4-ylboronic acid. The obtained compound was identified by MS/FAB and ¹H NMR.

C₄₂H₂₉F₂N₃ cal. 613.23, found 613.22.

### Synthesis Example 5: Synthesis of Compound 31

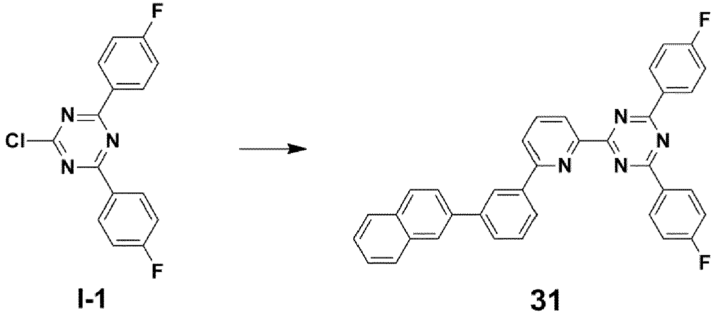

3.03 g (10 mmol) of Intermediate I-1, 3.25 g (10 mmol) of (6-(3-(naphthalen-2-yl)phenyl)pyridin-2-yl)boronic acid, 0.58 g (0.5 mmol) of Pd(PPh₃)₄, and 4.14 g (30 mmol) of K₂CO₃ were dissolved in 60 mL of a mixed solution including THF and H₂O (2/1) and stirred at a temperature of 80 °C for 16 hours. Then, the reaction solution was cooled to room temperature, and an organic layer was extracted therefrom three times by using 40 mL of water and 50 mL of ethylether. The extracted organic layer was dried by using magnesium sulfate, and a solvent was evaporated therefrom. Then, the residue obtained therefrom was separated and purified by silica gel column chromatography to obtain 3.12 g (yield: 57%) of Compound 31. The obtained compound was identified by MS/FAB and ¹H NMR.

C₃₆H₂₂F₂N₄ cal. 548.18, found 548.19.

### Synthesis Example 6: Synthesis of Compound 40

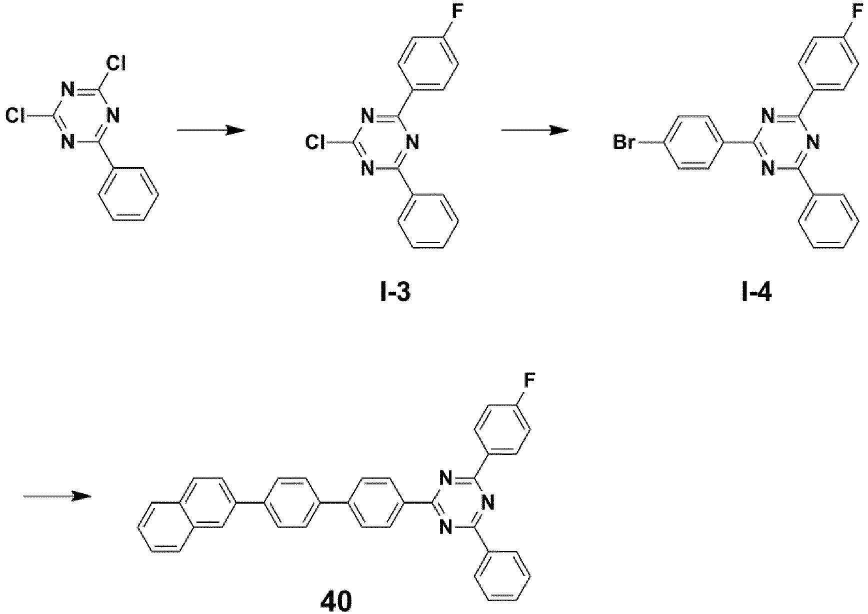

### Synthesis of Intermediate I-3

2.25 g (10 mmol) of 2,4-dichloro-6-phenyl-1,3,5-triazine was dissolved in 30 mL of THF, 10 mL of 4-fluorophenyl magnesium bromide (1.0 M in THF) was added thereto at a temperature of 0 °C, and the reaction solution was stirred at a temperature of 35 °C for 12 hours. Then, the reaction solution was cooled to room temperature, and an organic layer was extracted therefrom three times by using 60 mL of water and 60 mL of diethylether. The extracted organic layer was dried by using magnesium sulfate, and a solvent was evaporated therefrom. Then, the residue obtained therefrom was separated and purified by silica gel column chromatography to obtain 1.31 g (yield: 46%) of Intermediate I-3. The obtained compound was identified by LC-MS.

C₁₅H₉ClFN₃: M+1 285.1.

### Synthesis of Intermediate I-4

2.03 g (yield: 50%) of Intermediate I-4 was synthesized in substantially the same manner as in Synthesis of Intermediate I-2, except that Intermediate I-3 was used instead of Intermediate I-1. The obtained compound was identified by LC-MS.

C₂₁H₁₃BrFN₃: M+1 405.0.

### Synthesis of Compound 40

4.05 g (10 mmol) of Intermediate I-4, 2.48 g (10 mmol) of (4-(naphthalen-2-yl)phenyl)boronic acid, 0.58 g (0.5 mmol) of Pd(PPh₃)₄, and 4.14 g (30 mmol) of K₂CO₃ were dissolved in 60 mL of a mixed solution including THF and H₂O (2/1) and stirred at a temperature of 80 °C for 16 hours. The reaction solution was cooled to room temperature, and an organic layer was extracted therefrom three times by using 40 mL of water and 50 mL of ethylether. The extracted organic layer was dried by using magnesium sulfate, and a solvent was evaporated therefrom. Then, the residue obtained therefrom was separated and purified by silica gel column chromatography to obtain 4.07 g (yield: 77%) of Compound 40. The obtained compound was identified by MS/FAB and ¹H NMR.

C₃₇H₂₄FN₃ cal. 529.20, found 529.21.

### Synthesis Example 7: Synthesis of Compound 45

4.36 g (yield: 70%) of Compound 45 was synthesized in substantially the same manner as in Synthesis of Compound 2, except that 3-fluorophenyl magnesium bromide was used instead of 4-fluorophenyl magnesium bromide, and (4'-(naphthalen-2-yl)-[1,1'-biphenyl]-4-yl)boronic acid was used instead of [1,1'-biphenyl]-4-ylboronic acid. The obtained compound was identified by MS/FAB and ¹H NMR.

C₄₃H₂₇F₂N₃ cal. 623.22, found 623.22.

### Synthesis Example 8: Synthesis of Compound 60

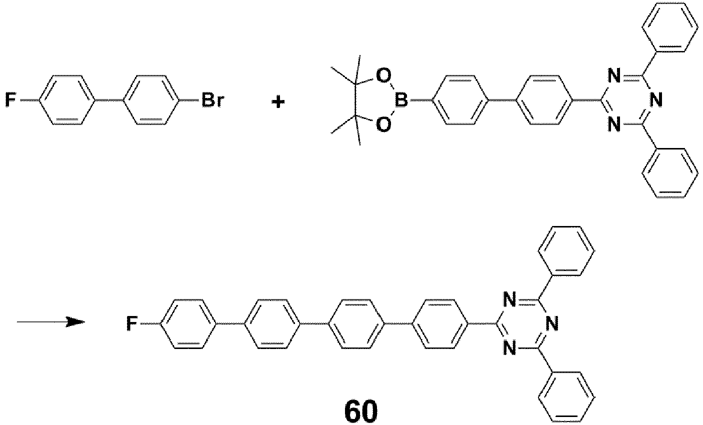

2.50 g (10 mmol) of 4-bromo-4'-fluoro-1,1'-biphenyl, 5.11 g (10 mmol) of 2,4-diphenyl-6-(4'-(4,4,5,5-tetramethyl-1,3,2-dioxaborolan-2-yl)-[1,1'-biphenyl]-4-yl)-1,3,5-triazine, 0.58 g (0.5 mmol) of Pd(PPh₃)₄, and 4.14 g (30 mmol) of K₂CO₃ were dissolved in 60 mL of a mixed solution including THF and H₂O (2/1) and stirred at a temperature of 80 °C for 16 hours. Then, the reaction solution was cooled to room temperature, and an organic layer was extracted therefrom three times by using 40 mL of water and 50 mL of ethylether. The extracted organic layer was dried by using magnesium sulfate, and a solvent was evaporated therefrom. Then, the residue obtained therefrom was separated and purified by silica gel column chromatography to obtain 4.39 g (yield: 79%) of Compound 60. The obtained compound was identified by MS/FAB and ¹H NMR.

C₃₉H₂₆FN₃ cal. 555.21, found 555.20.

### Synthesis Example 9: Synthesis of Compound 74

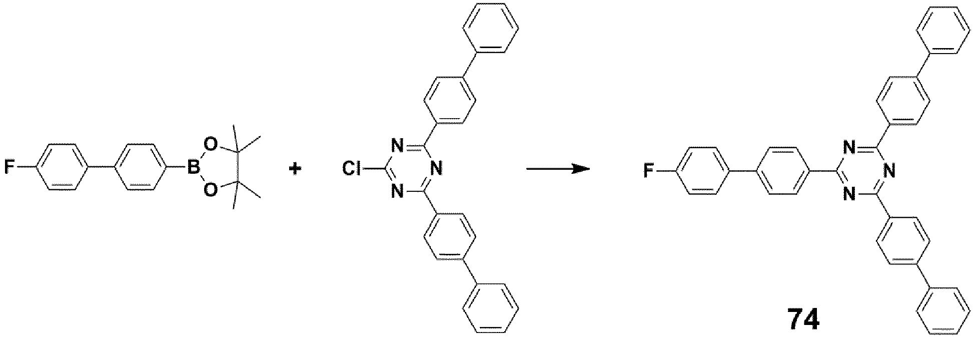

2.98 g (10 mmol) of 2-(4'-fluoro-[1,1'-biphenyl]-4-yl)-4,4,5,5-tetramethyl-1,3,2-dioxaborolane, 4.19 g (10 mmol) of 2,4-di([1,1'-biphenyl]-4-yl)-6-chloro-1,3,5-triazine, 0.58 g (0.5 mmol) of Pd(PPh₃)₄, and 4.14 g (30 mmol) of K₂CO₃ were dissolved in 60 mL of a mixed solution including THF and H₂O (2/1) and stirred at a temperature of 80 °C for 16 hours. Then, the reaction solution was cooled to room temperature, and an organic layer was extracted therefrom three times by using 40 mL of water and 50 mL of ethylether. The extracted organic layer was dried by using magnesium sulfate, and a solvent was evaporated therefrom. Then, the residue obtained therefrom was separated and purified by silica gel column chromatography to obtain 4.22 g (yield: 76%) of Compound 74. The obtained compound was identified by MS/FAB and ¹H NMR.

C₃₉H₂₆FN₃ cal. 555.21, found 555.20.

### Synthesis Example 10: Synthesis of Compound 81

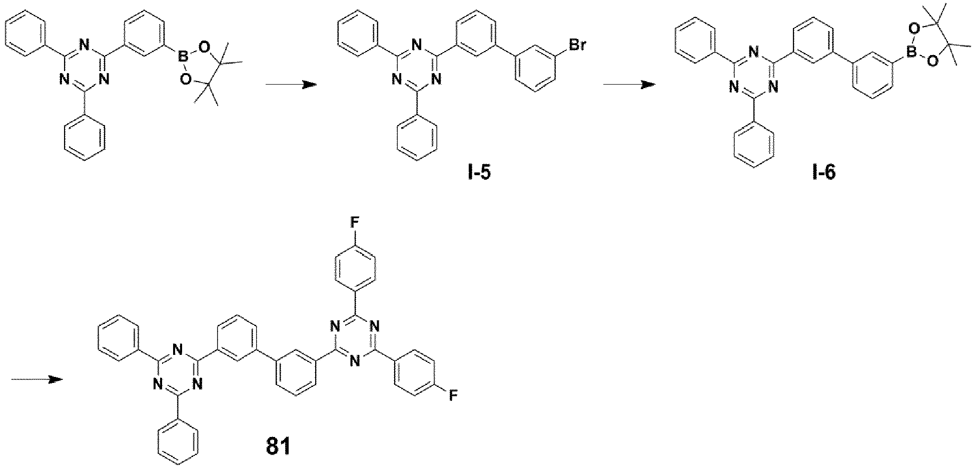

### Synthesis of Intermediate I-5

4.35 g (10 mmol) of 2,4-diphenyl-6-(3-(4,4,5,5-tetramethyl-1,3,2-dioxaborolan-2-yl)phenyl)-1,3,5-triazine, 3.38 g (12 mmol) of 1-bromo-4-iodobenzene, 0.58 g (0.5 mmol) of Pd(PPh₃)₄, and 4.14 g (30 mmol) of K₂CO₃ were dissolved in 60 mL of a mixed solution including THF and H₂O (2/1) and stirred at a temperature of 80 °C for 16 hours. Then, the reaction solution was cooled to room temperature, and an organic layer was extracted therefrom three times by using 40 mL of water and 50 mL of ethylether. Then extracted organic layer was dried by using magnesium sulfate, and a solvent was evaporated therefrom. Then, the residue obtained therefrom was separated and purified by silica gel column chromatography to obtain 3.38 g (yield: 73%) of Intermediate I-5. The obtained compound was identified by LC-MS.

C₂₇H₁₈BrN₃: M+1 463.1.

### Synthesis of Intermediate I-6

4.36 g (10 mmol) of Intermediate I-5, 2.54 g (10 mmol) of bis(pinacolato)diboron, 0.35 g (0.5 mmol) of Pd(PPh₃)₂Cl₂, and 2.94 g (30 mmol) of KOAc were dissolved in 60 mL of toluene and stirred at a temperature of 100 °C for 16 hours. The reaction solution was cooled to room temperature, and an organic layer was extracted therefrom three times by using 40 mL of water and 50 mL of ethylether. The extracted organic layer was dried by using magnesium sulfate, and a solvent was evaporated therefrom. Then, the residue obtained therefrom was separated and purified by silica gel column chromatography to obtain 3.63 g (yield: 71%) of Intermediate I-6. The obtained compound was identified by LC-MS.

C₃₃H₃₀BN₃O₂: M+1 511.2.

### Synthesis of Compound 81

3.03 g (10 mmol) of Intermediate I-1, 5.11 g (10 mmol) of Intermediate I-6, 0.58 g (0.5 mmol) of Pd(PPh₃)₄, and 4.14 g (30 mmol) of K₂CO₃ were dissolved in 60 mL of a mixed solution including THF and H₂O (2/1) and stirred at a temperature of 80 °C for 16 hours. Then, the reaction solution was cooled to room temperature, and an organic layer was extracted therefrom three times by using 40 mL of water and 50 mL of ethylether. The extracted organic layer was dried by using magnesium sulfate, and a solvent was evaporated therefrom. Then, the residue obtained therefrom was separated and purified by silica gel column chromatography to obtain 5.15 g (yield: 79%) of Compound 81. The obtained compound was identified by MS/FAB and ¹H NMR.

C₄₂H₂₆F₂N₆ cal. 625.22, found 625.23.

### Synthesis Example 11: Synthesis of Compound 90

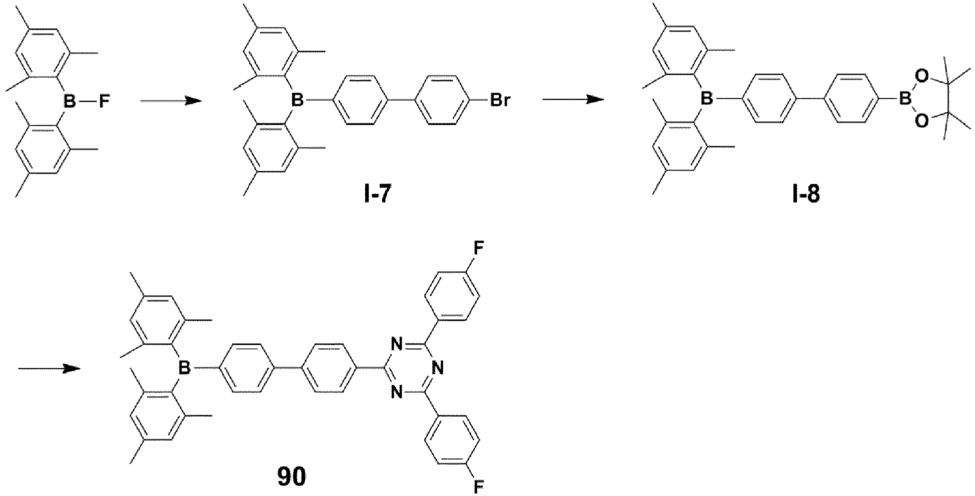

### Synthesis of Intermediate I-7

4.35 g (10 mmol) of 2,4-diphenyl-6-(3-(4,4,5,5-tetramethyl-1,3,2-dioxaborolan-2-yl)phenyl)-1,3,5-triazine, 3.38 g (12 mmol) of 1-bromo-4-iodobenzene, 0.58 g (0.5 mmol) of Pd(PPh₃)₄, and 4.14 g (30 mmol) of K₂CO₃ were dissolved in 60 mL of a mixed solution including THF and H₂O (2/1) and stirred at a temperature of 80 °C for 16 hours. Then, the reaction solution was cooled to room temperature, and an organic layer was extracted therefrom three times by using 40 mL of water and 50 mL of ethylether. The extracted organic layer was dried by using magnesium sulfate, and a solvent was evaporated therefrom. Then, the residue obtained therefrom was separated and purified by silica gel column chromatography to obtain 3.38 g (yield: 73%) of Intermediate I-5. The obtained compound was identified by LC-MS.

C₃₃H₃₀BN₃O₂: M+1 511.2.

### Synthesis of Intermediate I-8

4.80 g (10 mmol) of Intermediate I-7, 2.54 g (10 mmol) of bis(pinacolato)diboron, 0.35 g (0.5 mmol) of Pd(PPh₃)₂Cl₂, and 2.94 g (30 mmol) of KOAc were dissolved in 60 mL of toluene and stirred at a temperature of 100 °C for 16 hours. Then, the reaction solution was cooled to room temperature, and an organic layer was extracted therefrom three times by using 40 mL of water and 50 mL of ethylether. The extracted organic layer was dried by using magnesium sulfate, and a solvent was evaporated therefrom. Then, the residue obtained therefrom was separated and purified by silica gel column chromatography to obtain 3.70 g (yield: 70%) of Intermediate I-8. The obtained compound was identified by LC-MS.

C₃₆H₄₂B₂O₂: M+1 528.3.

### Synthesis of Compound 90

5.28 g (10 mmol) of Intermediate I-8, 3.03 g (10 mmol) of 2-chloro-4,6-bis(4-fluorophenyl)-1,3,5-triazine, 0.58 g (0.5 mmol) of Pd(PPh₃)₄, and 4.14 g (30 mmol) of K₂CO₃ were dissolved in 60 mL of a mixed solution including THF and H₂O (2/1) and stirred at a temperature of 80 °C for 16 hours. Then, the reaction solution was cooled to room temperature, and an organic layer was extracted therefrom three times by using 40 mL of water and 50 mL of ethylether. The extracted organic layer was dried by using magnesium sulfate, and a solvent was evaporated therefrom. Then, the residue obtained therefrom was separated and purified by silica gel column chromatography to obtain 4.89 g (yield: 73%) of Compound 90. The obtained compound was identified by MS/FAB and ¹H NMR.

C₄₅H₃₈BF₂N₃ cal. 669.31, found 669.30.

¹H NMR and MS/FAB of the synthesized Compounds are shown in Table 1. Methods of synthesizing compounds other than Compounds shown in Table 1 should be readily apparent to those of ordinary skill in the art by referring to the synthesis mechanisms and source materials described above.

**Table 1**

| Compound | ¹H NMR (CDCl₃, 400 MHz) | MS/FAB | |
|---|---|---|---|
| | | found | calc. |
| 2 | δ= 8.49-8.46 (m, 2H), 8.35-8.30 (m, 4H), 7.96-7.93 (M, 2H), 7.72-7.65 (m, 4H), 7.61-7.58 (m, 2H), 7.53-7.49 (m, 2H), 7.42-7.39 (m, 1H), 7.30-7.24 (m, 4H) | 497.16 | 497.17 |
| 5 | δ= 8.48-8.45 (m, 2H), 8.35-8.31 (m, 4H), 8.05-8.01 (m, 2H), 7.96-7.93 (m, 2H), 7.52-7.78 (m, 2H), 7.74-7.58 (m, 6H), 7.49-7.44 (m, 2H), 7.41-7.36 (m, 1H), 7.30-7.24 (m, 4H), 7.01-6.96 (m, 2H) | 623.23 | 623.22 |
| 9 | δ= 8.53-8.50 (m, 2H), 8.35-8.31 (m, 4H), 8.11 (dd, 2H), 7.97-7.91 (m, 4H), 7.72-7.66 (m, 6H), 7.62-7.58 (m, 2H), 7.53-7.49 (m, 2H), 7.42-7.38 (m, 1H), 7.30-7.24 (m, 4H) | 623.21 | 623.22 |
| 25 | δ= 8.52-8.48 (m, 2H), 8.35-8.30 (m, 4H), 7.91-7.87 (m, 2H), 7.76-7.71 (m, 2H), 7.63-7.60 (m, 2H), 7.55-7.47 (m, 4H), 7.42-7.38 (m, 1H), 7.31-7.24 (m, 6H), 1.56 (s, 6H) | 613.22 | 613.23 |
| 31 | δ= 8.56-8.52 (m, 2H), 8.43-8.38 (m, 4H), 8.19-8.17 (m, 1H), 8.07-8.05 (m, 1H), 8.00 (t, 1H), 7.93-7.91 (m, 1H), 7.89-7.77 (m, 4H), 7.72-7.69 (m, 1H), 7.62-7.49 (m, 2H), 7.34-7.24 (m, 5H) | 548.19 | 548.18 |
| 40 | δ= 8.81-8.78 (m, 2H), 8.49-8.46 (m, 2H), 8.35-8.30 (m, 2H), 8.09 (dd, 1H), 7.96-7.85 (m, 5H), 7.77-7.49 (m, 9H), 7.42-7.39 (m, 1H), 7.30-7.25 (m, 2H) | 529.21 | 529.20 |
| 45 | δ= 8.81-8.78 (m, 2H), 8.49-8.46 (m, 2H), 8.35-8.30 (m, 2H), 8.10-8.08 (m, 1H), 7.96-7.85 (m, 5H), 7.77-7.49 (m, 9H), 7.42-7.39 (m, 1H), 7.30-7.25 (m, 2H) | 623.22 | 623.22 |
| 60 | δ= 8.81-8.78 (m, 4H), 8.49-8.46 (m, 2H), 7.96-7.93 (m, 2H), 7.76-7.59 (m, 12H), 7.42-7.38 (m, 2H), 7.18-7.13 (m, 2H), 6.71-6.65 (m, 2H) | 555.20 | 555.21 |
| 74 | δ= 8.49-8.43 (m, 6H), 8.00-7.92 (m, 6H), 7.60-7.58 (m, 4H), 7.52-7.49 (m, 4H), 7.42-7.39 (m, 2H), 7.18-7.13 (m, 2H), 6.71-6.65 (m, 2H) | 555.20 | 555.21 |
| 81 | δ= 8.80-8.78 (m, 4H), 8.65-8.64 (m, 2H), 8.59-8.56 (m, 2H), 8.35-8.30 (m, 4H), 7.86-7.83 (m, 2H), 7.63-7.52 (m, 6H), 7.42-7.38 (m, 2H), 7.30-7.24 (m, 4H) | 625.23 | 625.22 |
| 90 | δ= 8.49-8.46 (m, 2H), 8.35-8.30 (m, 4H), 7.97-7.93 (m, 2H), 7.67-7.64 (m, 2H), 7.56-7.53 (m, 2H), 7.30-7.24 (m, 4H), 6.80-6.77 (m, 4H), 2.19 (s, 18H) | 669.30 | 669.31 |

Methods of synthesizing compounds other than the Compounds synthesized according to Synthesis Examples 1 to 11 may also be readily apparent to those of ordinary skill in the art by referring to the synthesis mechanisms and source materials described above.

### Example 1

As an anode, a Corning 15 Ω/cm² (1,200 Å) ITO glass substrate was cut to a size of 50 mm × 50 mm × 0.7 mm, sonicated with isopropyl alcohol and pure water each for 5 minutes, and then cleaned by exposure to ultraviolet rays and ozone for 30 minutes. Then, the resultant ITO glass substrate was provided to a vacuum deposition apparatus.

2-TNATA, which is a generally available material, was vacuum-deposited on the anode to form a hole injection layer having a thickness of 600Å, and 4,4'-bis[N-(1-naphthyl)-N-phenylamino]biphenyl (NPB), which is a generally available material, was vacuum-deposited as a hole transport compound on the hole injection layer to form a hole transport layer having a thickness of 300 Å.

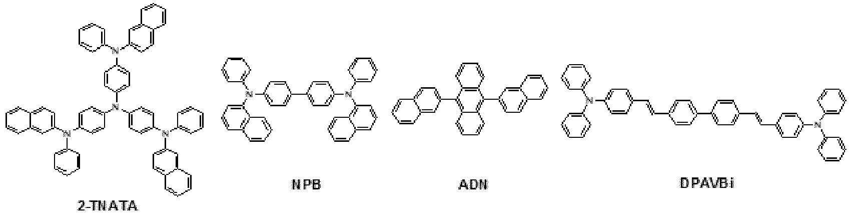

9,10-di-naphthalene-2-yl-anthracene (ADN) and 4,4'-bis[2-(4-(N,N-diphenylamino)phenyl)vinyl]biphenyl (DPAVBi), which are generally available compounds, were respectively co-deposited as a blue fluorescent host and a blue fluorescent dopant on the hole transport layer at a weight ratio of 98:2 to form an emission layer having a thickness of 300 Å.

Then, Compound 2 was deposited on the emission layer to form an electron transport layer having a thickness of 300 Å, LiF, which is an alkali metal halide, was deposited on the electron transport layer to form an electron injection layer having a thickness of 10 Å, and Al was vacuum-deposited on the electron injection layer to form a cathode electrode having a thickness of 3,000 Å, thereby forming a LiF/AI electrode. In this manner, an organic light-emitting device was manufactured.

### Example 2

An organic light-emitting device was manufactured in substantially the same manner as in Example 1, except that Compound 5 was used instead of Compound 2 in forming an electron transport layer.

### Example 3

An organic light-emitting device was manufactured in substantially the same manner as in Example 1, except that Compound 9 was used instead of Compound 2 in forming an electron transport layer.

### Example 4

An organic light-emitting device was manufactured in substantially the same manner as in Example 1, except that Compound 25 was used instead of Compound 2 in forming an electron transport layer.

### Example 5

An organic light-emitting device was manufactured in substantially the same manner as in Example 1, except that Compound 31 was used instead of Compound 2 in forming an electron transport layer.

### Example 6

An organic light-emitting device was manufactured in substantially the same manner as in Example 1, except that Compound 40 was used instead of Compound 2 in forming an electron transport layer.

### Example 7

An organic light-emitting device was manufactured in substantially the same manner as in Example 1, except that Compound 45 was used instead of Compound 2 in forming an electron transport layer.

### Example 8

An organic light-emitting device was manufactured in substantially the same manner as in Example 1, except that Compound 60 was used instead of Compound 2 in forming an electron transport layer.

### Example 9

An organic light-emitting device was manufactured in substantially the same manner as in Example 1, except that Compound 74 was used instead of Compound 2 in forming an electron transport layer.

### Example 10

An organic light-emitting device was manufactured in substantially the same manner as in Example 1, except that Compound 81 was used instead of Compound 2 in forming an electron transport layer.

### Example 11

An organic light-emitting device was manufactured in substantially the same manner as in Example 1, except that Compound 90 was used instead of Compound 2 in forming an electron transport layer.

### Comparative Example 1

An organic light-emitting device was manufactured in substantially the same manner as in Example 1, except that Compound 200 was used instead of Compound 2 in forming an electron transport layer.

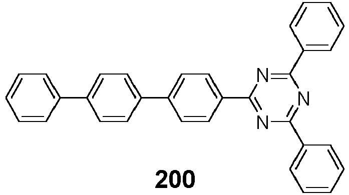

### Comparative Example 2

An organic light-emitting device was manufactured in substantially the same manner as in Example 1, except that Compound 201 was used instead of Compound 2 in forming an electron transport layer.

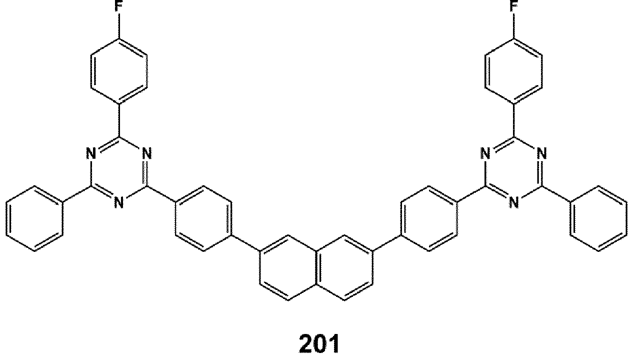

### Comparative Example 3

An organic light-emitting device was manufactured in substantially the same manner as in Example 1, except that Compound 202 was used instead of Compound 2 in forming an electron transport layer.

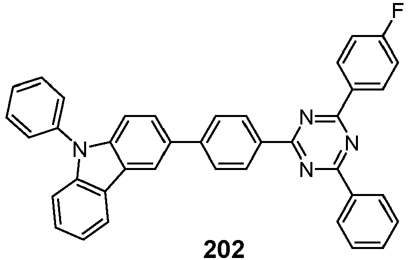

The driving voltage, luminance, efficiency (cd/A), and half lifespan of the organic light-emitting devices manufactured according to Examples 1 to 11 and Comparative Examples 1 to 3 were measured at a current density of 50 mA/cm², and results thereof are shown in Table 2 below.

**Table 2**

| | Material | Driving voltage (V) | Current density (mA/cm²) | Luminance (cd/m²) | Efficiency (cd/A) | Emission color | Half lifespan (hr @ 100mA/c m²) |
|---|---|---|---|---|---|---|---|
| Example 1 | Compound 2 | 3.65 | 50 | 3,705 | 7.41 | Blue | 710hr |
| Example 2 | Compound 5 | 3.63 | 50 | 3,890 | 7.78 | Blue | 706hr |
| Example 3 | Compound 9 | 3.60 | 50 | 3,775 | 7.55 | Blue | 721hr |
| Example 4 | Compound 25 | 3.69 | 50 | 3,750 | 7.50 | Blue | 759hr |
| Example 5 | Compound 31 | 3.62 | 50 | 3,790 | 7.58 | Blue | 732hr |
| Example 6 | Compound 40 | 3.64 | 50 | 3,820 | 7.64 | Blue | 745hr |
| Example 7 | Compound 45 | 3.60 | 50 | 3,815 | 7.63 | Blue | 720hr |
| Example 8 | Compound 60 | 3.55 | 50 | 3,785 | 7.57 | Blue | 726hr |
| Example 9 | Compound 74 | 3.58 | 50 | 3,795 | 7.59 | Blue | 733hr |
| Example 10 | Compound 81 | 3.61 | 50 | 3,800 | 7.60 | Blue | 750hr |
| Example 11 | Compound 90 | 3.59 | 50 | 3,810 | 7.62 | Blue | 738hr |
| Comparative Example 1 | Compound 200 | 3.85 | 50 | 3,555 | 7.11 | Blue | 633hr |
| Comparative Example 2 | Compound 201 | 3.90 | 50 | 3,570 | 7.14 | Blue | 656hr |
| Comparative Example 3 | Compound 202 | 4.32 | 50 | 3,500 | 7.00 | Blue | 602hr |

From Table 2, when the compound according to one or more embodiments was used as an electron transport material, excellent I-V-L characteristics including a low driving voltage and remarkably improved efficiency and luminance were exhibited, as compared with Compounds 201 to 202 as well as Compound 200, which are generally available materials. For example, an excellent lifespan improvement effect was exhibited.

For example, when the compound according to one or more embodiments is used as an electron transport material of a device, excellent effects may be exhibited in terms of driving voltage, luminance, efficiency, and lifespan. Compounds 2, 5, 9, 25, 31, 40, 45, 60, 74, 81, and 90 are shown below.

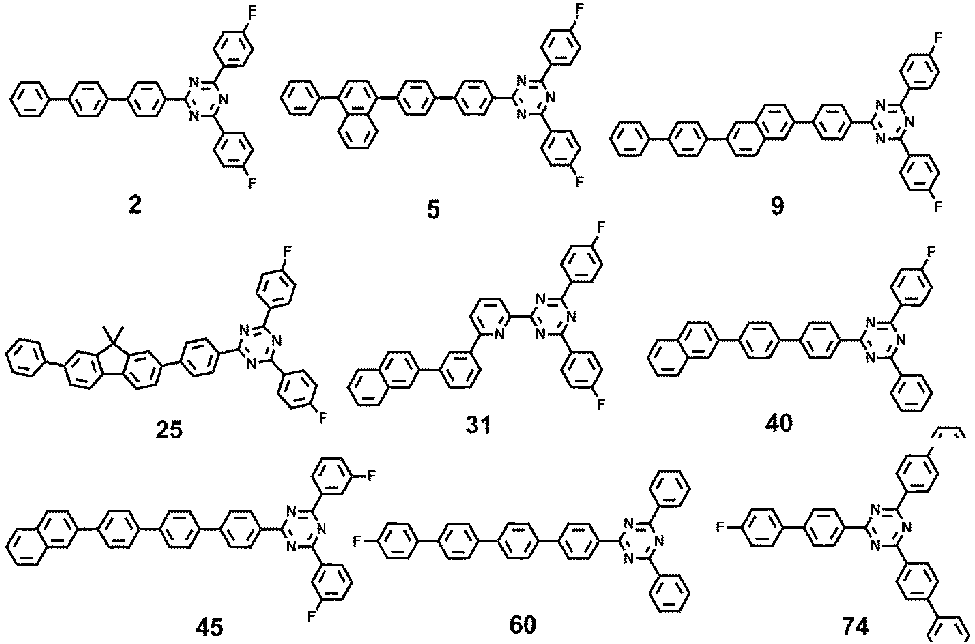

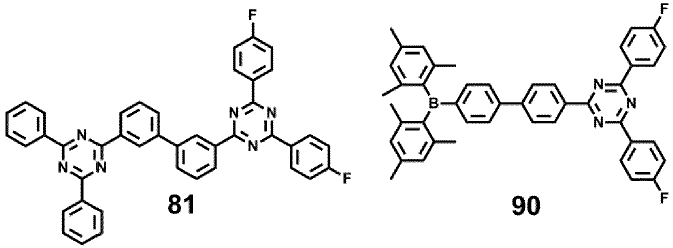

An organic light-emitting device according to an embodiment may have a low driving voltage, high efficiency, and a long lifespan.

It will be understood that, although the terms "first," "second," "third," etc., may be used herein to describe various elements, components, regions, layers and/or sections, these elements, components, regions, layers and/or sections should not be limited by these terms. These terms are used to distinguish one element, component, region, layer or section from another element, component, region, layer or section. Thus, a first element, component, region, layer or section described below could be termed a second element, component, region, layer or section.

Spatially relative terms, such as "beneath," "below," "lower," "under," "above," "upper," and the like, may be used herein for ease of explanation to describe one element or feature's relationship to another element(s) or feature(s) as illustrated in the figures. It will be understood that the spatially relative terms are intended to encompass different orientations of the device in use or in operation, in addition to the orientation depicted in the figures. For example, if the device in the figures is turned over, elements described as "below" or "beneath" or "under" other elements or features would then be oriented "above" the other elements or features. Thus, the example terms "below" and "under" can encompass both an orientation of above and below. The device may be otherwise oriented (e.g., rotated 90 degrees or at other orientations) and the spatially relative descriptors used herein should be interpreted accordingly.

It will be understood that when an element or layer is referred to as being "on," "connected to," or "coupled to" another element or layer, it can be directly on, connected to, or coupled to the other element or layer, or one or more intervening elements or layers may be present. In addition, it will also be understood that when an element or layer is referred to as being "between" two elements or layers, it can be the only element or layer between the two elements or layers, or one or more intervening elements or layers may also be present.

The terminology used herein is for the purpose of describing particular embodiments only and is not intended to be limiting of the present disclosure. As used herein, the singular forms "a" and "an" are intended to include the plural forms as well, unless the context clearly indicates otherwise. It will be further understood that the terms "comprises," "comprising," "includes," and "including," when used in this specification, specify the presence of the stated features, integers, acts, operations, elements, and/or components, but do not preclude the presence or addition of one or more other features, integers, acts, operations, elements, components, and/or groups thereof.

As used herein, the terms "substantially," "about," and similar terms are used as terms of approximation and not as terms of degree, and are intended to account for the inherent deviations in measured or calculated values that would be recognized by those of ordinary skill in the art. Further, the use of "may" when describing embodiments of the present disclosure refers to "one or more embodiments of the present disclosure." As used herein, the terms "use," "using," and "used" may be considered synonymous with the terms "utilize," "utilizing," and "utilized," respectively.

Also, any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein, and any minimum numerical limitation recited in this specification is intended to include all higher numerical limitations subsumed therein.

## Claims

1. A heterocyclic compound selected from Compounds 2, 5, 9, 25, 31, 40, 45, 60, 74, 81, and 90:

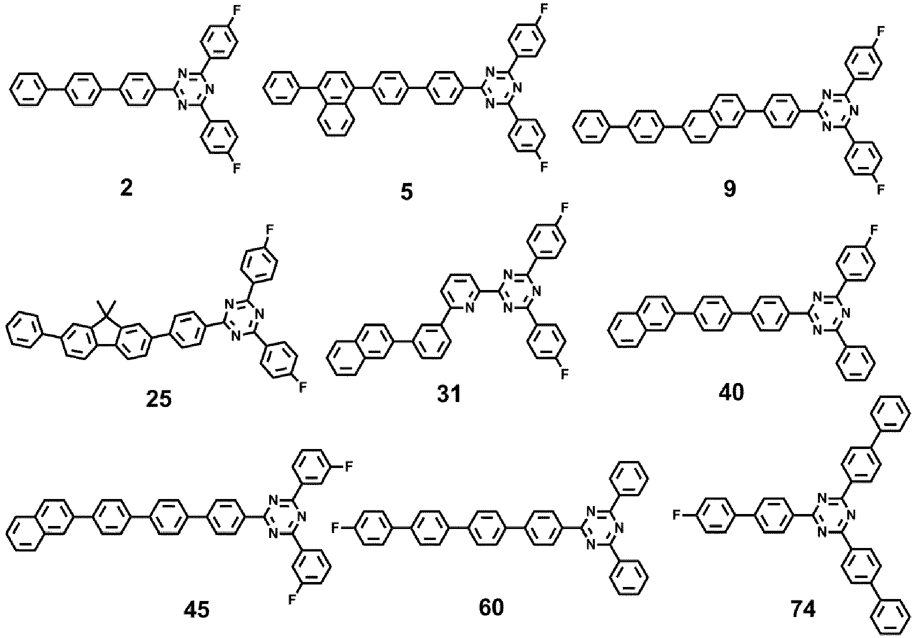

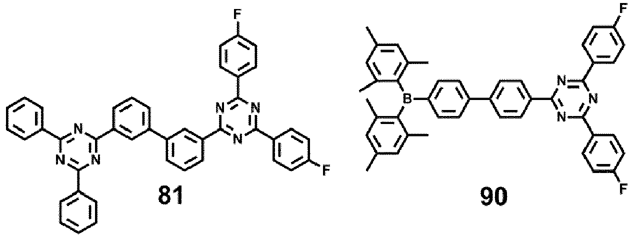

2. An organic light-emitting device comprising:
a first electrode;
a second electrode facing the first electrode; and
an organic layer between the first electrode and the second electrode, wherein the organic layer comprises an emission layer and at least one of the heterocyclic compounds of claim 1.

3. The organic light-emitting device of claim 2, wherein:
the first electrode is an anode,
the second electrode is a cathode,
the organic layer further comprises a hole transport region between the first electrode and the emission layer and an electron transport region between the emission layer and the second electrode, wherein
the hole transport region preferably comprises a hole injection layer, a hole transport layer, an emission auxiliary layer, an electron blocking layer and/or any combination thereof, and/or
the electron transport region preferably comprises a buffer layer, a hole blocking layer, an emission auxiliary layer, an electron control layer, an electron transport layer, an electron injection layer, and/or any combination thereof.

4. The organic light-emitting device of claim 3, wherein:
the electron transport region comprises the heterocyclic compound, an electron transport layer which includes the heterocyclic compound, and/or an emission auxiliary layer, wherein the emission auxiliary layer preferably comprises the heterocyclic compound.

5. The organic light-emitting device of claim 3, wherein:
the hole transport region comprises a p-dopant, wherein
the p-dopant has a lowest unoccupied molecular orbital (LUMO) energy level of -3.5 eV or less, and preferably comprises a cyano group-containing compound.

6. The organic light-emitting device of claim 2, wherein:
the emission layer is a first emission layer for emitting first color light,
the organic light-emitting device further comprises, between the first electrode and the second electrode, i) at least one second emission layer for emitting second color light or ii) at least one second emission layer for emitting second color light and at least one third emission layer for emitting third color light,
a maximum emission wavelength of the first color light, a maximum emission wavelength of the second color light, and a maximum emission wavelength of the third color light are identical to or different from one another, and
the first color light and the second color light are emitted in the form of mixed light, or the first color light, the second color light, and the third color light are emitted in the form of mixed light.

## Patentansprüche

1. Heterocyclische Verbindung, ausgewählt aus den Verbindungen 2, 5, 9, 25, 31, 40, 45, 60, 74, 81 und 90:

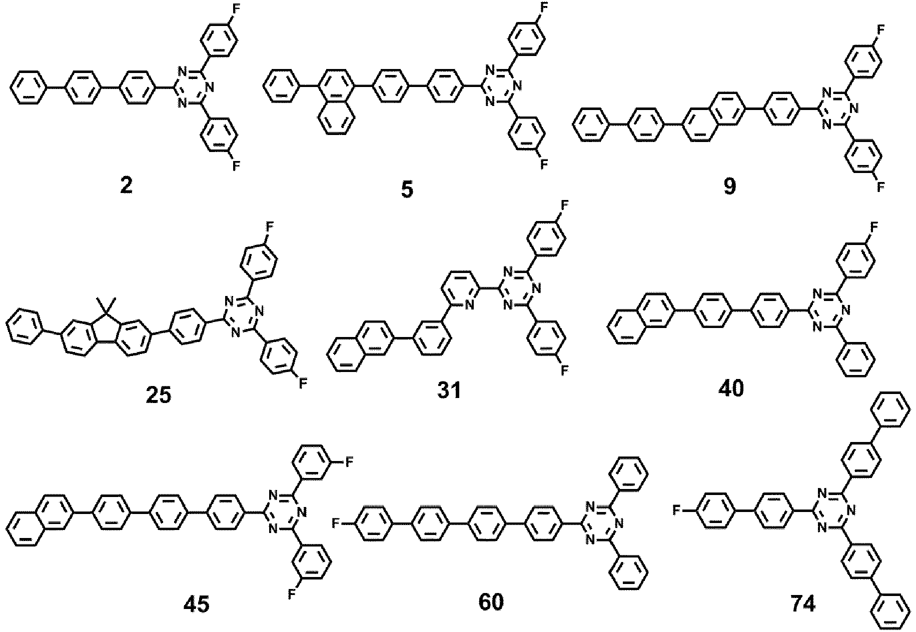

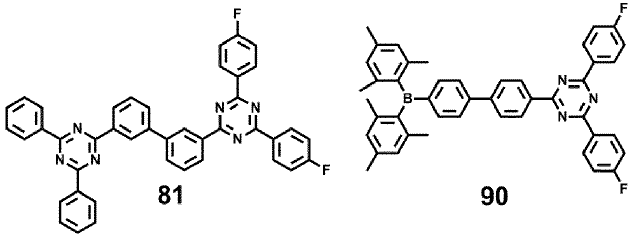

2. Organische lichtemittierende Vorrichtung, die Folgendes umfasst:
eine erste Elektrode;
eine zweite Elektrode, die der ersten Elektrode gegenüberliegt; und
eine organische Schicht zwischen der ersten Elektrode und der zweiten Elektrode, wobei die organische Schicht eine Emissionsschicht und mindestens eine der heterocyclischen Verbindungen nach Anspruch 1 umfasst.

3. Organische lichtemittierende Vorrichtung nach Anspruch 2, wobei:
die erste Elektrode ist eine Anode,
die zweite Elektrode ist eine Kathode,
die organische Schicht ferner einen Lochtransportbereich zwischen der ersten Elektrode und der Emissionsschicht und einen Elektronentransportbereich zwischen der Emissionsschicht und der zweiten Elektrode umfasst, wobei
der Lochtransportbereich umfasst vorzugsweise eine Lochinjektionsschicht, eine Lochtransportschicht, eine Emissionshilfsschicht, eine Elektronensperrschicht und/oder eine beliebige Kombination davon, und/oder
der Elektronentransportbereich umfasst vorzugsweise eine Pufferschicht, eine Lochsperrschicht, eine Elektronenkontrollschicht, eine Emissionshilfsschicht, eine Elektronentransportschicht, eine Elektroneninjektionsschicht und/oder eine beliebige Kombination davon.

4. Organische lichtemittierende Vorrichtung nach Anspruch 3, wobei:
der Elektronentransportbereich die heterocyclische Verbindung, eine Elektronentransportschicht, die die heterocyclische Verbindung enthält, und/oder eine Emissionshilfsschicht umfasst, wobei die Emissionshilfsschicht vorzugsweise die heterocyclische Verbindung umfasst.

5. Organische lichtemittierende Vorrichtung nach Anspruch 3, wobei:
der Lochtransportbereich einen p-Dotierstoff enthält, wobei
der p-Dotierstoff hat ein Energieniveau des niedrigsten unbesetzten Molekülorbitals (LUMO) von -3,5 eV oder weniger und umfasst vorzugsweise eine Cyanogruppe enthaltende Verbindung.

6. Organische lichtemittierende Vorrichtung nach Anspruch 2, wobei:
die Emissionsschicht ist eine erste Emissionsschicht zur Emission von erstem Farblicht,
die organische lichtemittierende Vorrichtung ferner zwischen der ersten Elektrode und der zweiten Elektrode i) mindestens eine zweite Emissionsschicht zur Emission von Licht der zweiten Farbe oder ii) mindestens eine zweite Emissionsschicht zur Emission von Licht der zweiten Farbe und mindestens eine dritte Emissionsschicht zur Emission von Licht der dritten Farbe umfasst,
eine maximale Emissionswellenlänge des ersten Farblichts, eine maximale Emissionswellenlänge des zweiten Farblichts und eine maximale Emissionswellenlänge des dritten Farblichts identisch oder verschieden voneinander sind, und
das erste Farblicht und das zweite Farblicht in Form von Mischlicht emittiert werden, oder das erste Farblicht, das zweite Farblicht und das dritte Farblicht in Form von Mischlicht emittiert werden.

## Revendications

1. Composé hétérocyclique choisi parmi des composés 2, 5, 9, 25, 31, 40, 45, 60, 74, 81 et 90 :

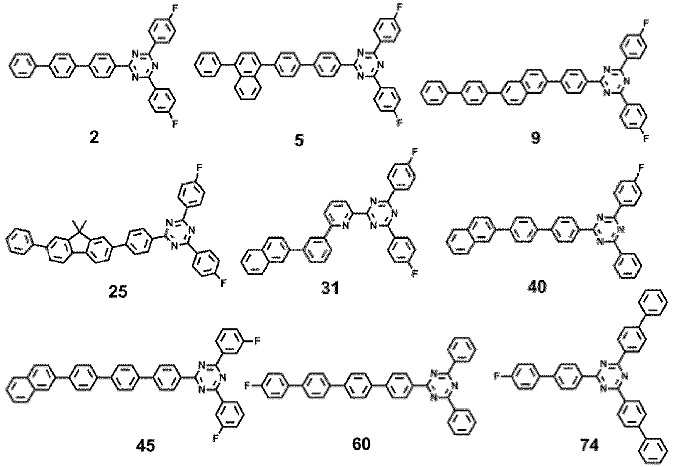

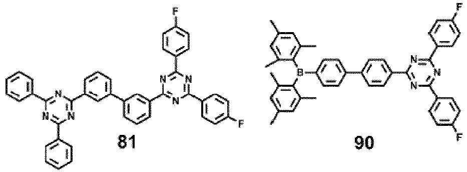

2. Dispositif électroluminescent organique comprenant :
une première électrode ;
une deuxième électrode tournée vers la première électrode ; et
une couche organique entre la première électrode et la deuxième électrode, dans lequel la couche organique comprend une couche d'émission et au moins un des composés hétérocycliques selon la revendication 1.

3. Dispositif électroluminescent organique selon la revendication 2, dans lequel :
la première électrode est une anode,
la deuxième électrode est une cathode,
la couche organique comprend en outre une région de transport de trous entre la première électrode et la couche d'émission et une zone de transport d'électrons entre la couche d'émission et la deuxième électrode, dans lequel
la région de transport de trous comprend de manière préférée une couche d'injection de trous, une couche de transport de trous, une couche auxiliaire d'émission, une couche de blocage d'électrons et/ou toute combinaison de celles-ci,
et/ou
la région de transport d'électrons comprend de manière préférée une couche tampon, une couche de blocage de trous, une couche auxiliaire d'émission, une couche de commande d'électrons, une couche de transport d'électrons, une couche d'injection d'électrons, et/ou toute combinaison de celles-ci .

4. Dispositif électroluminescent organique selon la revendication 3, dans lequel :
la région de transport d'électrons comprend le composé hétérocyclique, une couche de transport d'électrons qui comporte le composé hétérocyclique, et/ou une couche auxiliaire d'émission, dans lequel la couche auxiliaire d'émission comprend de manière préférée le composé hétérocyclique.

5. Dispositif électroluminescent organique selon la revendication 3, dans lequel :
la région de transport de trous comprend un p-dopant, dans lequel
le p-dopant présente un niveau d'énergie LUMO [lowest unoccupied molecular orbital - orbitale moléculaire non occupée la plus faible] inférieur ou égal à -3,5 eV et comprend de manière préférée un composant contenant un groupe cyano.

6. Dispositif électroluminescent organique selon la revendication 2, dans lequel :
la couche d'émission est une première couche d'émission destinée à émettre une première lumière colorée,
le dispositif électroluminescent organique comprend en outre, entre la première électrode et la deuxième électrode, i) au moins une deuxième couche d'émission destinée à émettre une deuxième lumière colorée ou ii) au moins une deuxième couche d'émission destinée à émettre une deuxième lumière colorée et au moins une troisième couche d'émission destinée à émettre une troisième lumière colorée,
une longueur d'onde d'émission maximale de la première lumière colorée, une longueur d'onde d'émission maximale de la deuxième lumière colorée, et une longueur d'onde d'émission maximale de la troisième lumière colorée sont identiques ou différentes les unes des autres, et
la première lumière colorée et la deuxième lumière colorée sont émises sous la forme d'une lumière mixte, ou la première lumière colorée, la deuxième lumière colorée et la troisième lumière colorée sont émises sous la forme d'une lumière mixte.
